# EUROPÄISCHE PATENTSCHRIFT

(11) **EP 3 421 470 B1**
(45) Veröffentlichungstag und Bekanntmachung des Hinweises auf die Patenterteilung: **20.01.2021**
(21) Anmeldenummer: 18178621.1
(22) Anmeldetag: 21.11.2012
(51) Int. Cl.: C07D 471/04, A61K 31/506, A61P 9/00

(54) **SUBSTITUIERTE 5-FLUOR-1H-PYRAZOLOPYRIDINE IN KRISTALLINER FORM**
SUBSTITUTED 5-FLUORO-1H-PYRAZOLOPYRIDINES IN CRYSTALLINE FORM
5-FLUORO-1H-PYRAZOLOPYRIDINES SUBSTITUÉES SOUS FORME CRISTALLINE

(30) Priorität: 25.11.2011 EP 11190789; 07.12.2011 EP 11192301
(43) Veröffentlichungstag der Anmeldung: 02.01.2019
(62) Teilanmeldung aus: 12790891.1
(73) Patentinhaber: Adverio Pharma GmbH, 12529 Schönefeld (DE)
(72) Erfinder: FEY, Peter, 42111 Wuppertal (DE); GRUNENBERG, Alfons, 42115 Wuppertal (DE); BIERER, Donald, 42781 Haan (DE)
(74) Vertreter: BIP Patents

(56) Entgegenhaltungen:
- WO-A1-03/095451
- WO-A1-2011/147809
- WO-A1-2012/028647
- WO-A2-2011/064156

## Beschreibung

Gegenstand der Offenbarung der vorliegenden Anmeldung ist ein neues und effizientes Verfahren zur Herstellung von neuen substituierten 5-Fluor-1H-pyrazolopyridinen der Formel (VI) 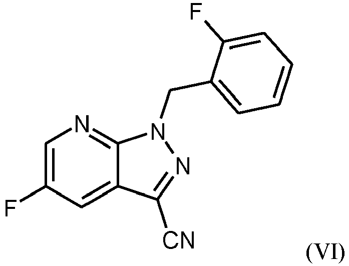 welche als Zwischenstufe zur Herstellung von Arzneimitteln und zur Herstellung von Arzneimitteln zur Behandlung und/oder Prophylaxe von kardiovaskulären Erkrankungen, dient.

Insbesondere eignen sich die 5-Fluor-1H-pyrazolopyridine der Formel (VI) zur Herstellung von Verbindung der Formel (I) 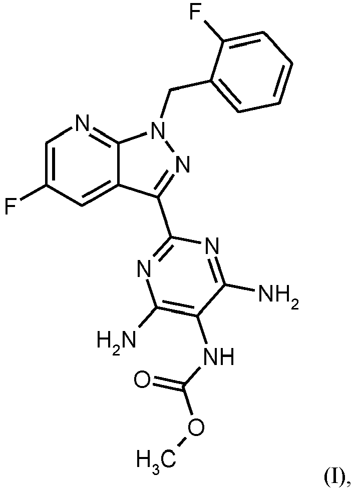 welche zur Herstellung von Arzneimitteln und zur Herstellung von Arzneimitteln zur Behandlung und/oder Prophylaxe von kardiovaskulären Erkrankungen, dient.

Die Verbindung der Formel (I) wirkt als Stimulator der löslichen Guanylatcyclase und kann als Mittel zur Prophylaxe und/oder Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, peripheren Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektiler Dysfunktion, weiblicher sexueller Dysfunktion, Osteoporose, Glaukom, pulmonaler Hypertonie, Gastroparese, Sklerodermie und Inkontinenz eingesetzt werden.

Die Verbindung der Formel (I) kann in verschiedenen Kristallformen und Solvaten vorliegen. Die Verbindung der Formel (I) existiert in fünf Modifikationen mit den Schmelzpunkten 257°C (Mod. I), 253°C (Mod. II), 247°C (Mod. III), 246°C (Mod. IV), 234°C (Mod. V), einem Dimethylformamid-Wasser-Solvat (DMF-Gehalt 13,6 %, Wasser-Gehalt 0,9 %), einem Di-Dimethylsulfoxid-Solvat (stöchiometrischer Wert: 26,8 % DMSO), einem Tri-Essigsäure-Solvat (29,7 % Acetat), einem Monohydrat (4,1 % Wasser) und einem Dihydrat (7,8 % Wasser). Im Stand der Technik, WO 2011/147809, ist die Verbindung der Formel (I) im Beispiel 1 als Substanz beschrieben.

Die Kristallmodifikation der Verbindung Formel (I) in der Modifikation (I) zeichnet sich durch eine Stabilität und insbesondere dadurch aus, dass sie auch im Mikronisierungsprozess stabil ist und somit keine Umwandlung und Rekristallisation stattfindet.

Das Di-Dimethylsulfoxid-Solvat der Verbindung der Formel (I) hat den Vorteil einer deutlich besseren Filtrierbarkeit als die Substanz im Stand der Technik. Weiterhin führt das Herstellverfahren über das Di-Dimethylsulfoxid-Solvat der Verbindung der Formel (I) zu einer sehr hohen Reinheit der Verbindung der Formel (I).

WO 03/095451, WO 2011/064156 und WO 2011/064171 offenbaren die Synthese von Pyrazolopyridinen, die am Pyridinring nicht substituiert sind. In diesen Offenbarungen wird das bicyclische Ringsystem durch Umsetzung von Phenylbenzylhydrazin mit Cyanobrenztraubensäureethylester aufgebaut. Diese Synthesemethode ist nicht für den Aufbau von 5-Fluor-1H-pyrazolopyridinen geeignet.

In WO 2009/018415 wird die Synthese von 5-Fluor-1H-pyrazolo[3,4-b]pyridin-3-amin E beschrieben. Durch selektive Dechlorierung der Nikotinsäure A zur Verbindung B, anschliessende Überführung in das Amid C, dessen Reduktion zum Nitril und die abschliessende Zyklisierung mit Hydrazinhydrat wird das 5-Fluor-1H-pyrazolo[3,4-b]pyridin-Core aufgebaut. Das folgende Schema 1 veranschaulicht die Synthese.

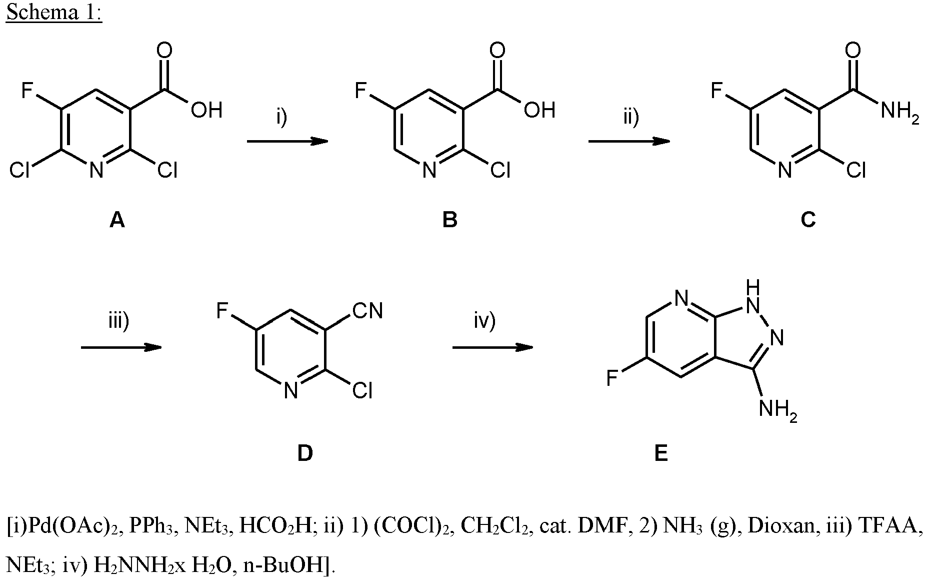

Nachteil dieses Verfahrens ist, dass ausgehend von 5-Fluor-1H-pyrazolo[3,4-b]pyridin E weitere Schritte wie die Diazotierungsreaktion und Umsetzung zur Iodo-Verbindung gefolgt von einer Alkylierung mit einem Benzylderivat und anschließender Funktionalisierung zur Einführung der Cyanogruppe erforderlich sind, um die gewünschten 5-Fluor-1H-Pyrazolopyridine der Formel (VI) zu erhalten. Dies wird in Schema 2 beispielhaft verdeutlicht.

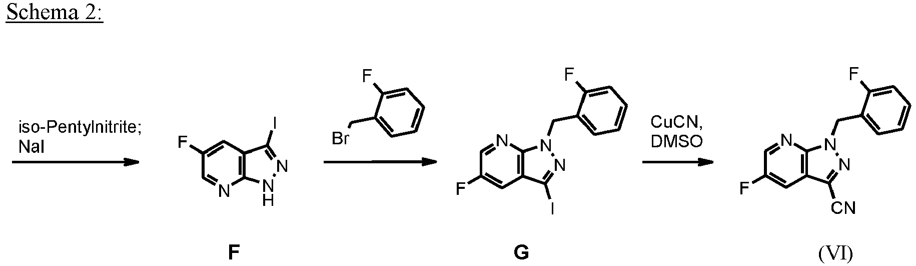

Ein weiterer Nachteil ist, dass die Diazotierung wasserfrei durchgeführt wird und das Diazoniumsalz isoliert werden muss, was bei der Übertragung in den technischen Maßstab erhebliche Sicherheitsvorkehrungen erforderlich macht und somit hohe Herstellungskosten verursacht.

Ein weiterer Nachteil ist, dass die Alkylierung mit einem Benzylderivat unselektiv verläuft und das Produkt nach aufwändiger Reinigung und Trennung der Isomere in nur geringer Ausbeute erhalten wird.

Ein weiterer Nachteil ist, dass bei der Cyanierung giftiges Kupfercyanid gehandhabt werden muss, was zusätzliche Sicherheitsvorkehrungen bei der Herstellung sowie bei der Entsorgung von Mutterlaugen und wässrigen Phasen erforderlich macht und somit hohe Herstellungskosten verursacht.

Ein weiterer Nachteil ist, dass die Herstellung von 5-Fluor-1H-Pyrazolopyridinen der Formel (VI) nach dem in Schema 1 beschriebenen Verfahren die Herstellung und Reinigung von sieben Intermediaten erfordert und nur eine geringe Gesamtausbeute liefert.

Gegenstand der Offenbarung ist ein effizientes Verfahren mit hoher Ausbeute zur Herstellung von 5-Fluor-1H-Pyrazolopyridinen der Formel (VI) 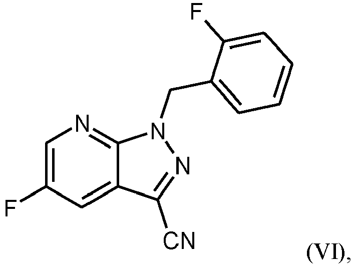 als Schlüsselbaustein für ein effizientes Verfahren mit hoher Ausbeute zur Herstellung von Verbindung der Formel (I) 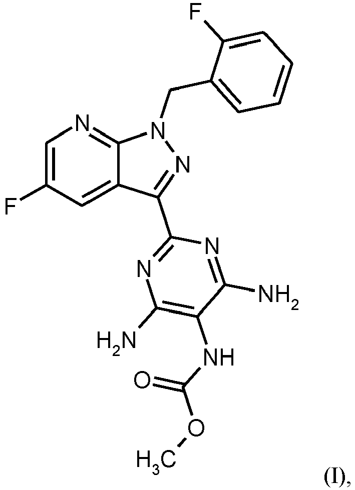 sowie dessen *N*-Oxide Salze, Solvate, Salze der *N*-Oxide und Solvate der *N*-Oxide und Salze.

Dieser Gegenstand der Offenbarung wird wie folgt, gelöst. Das folgende Schema 3 veranschaulicht exemplarisch die einzelnen Reaktionsschritte.

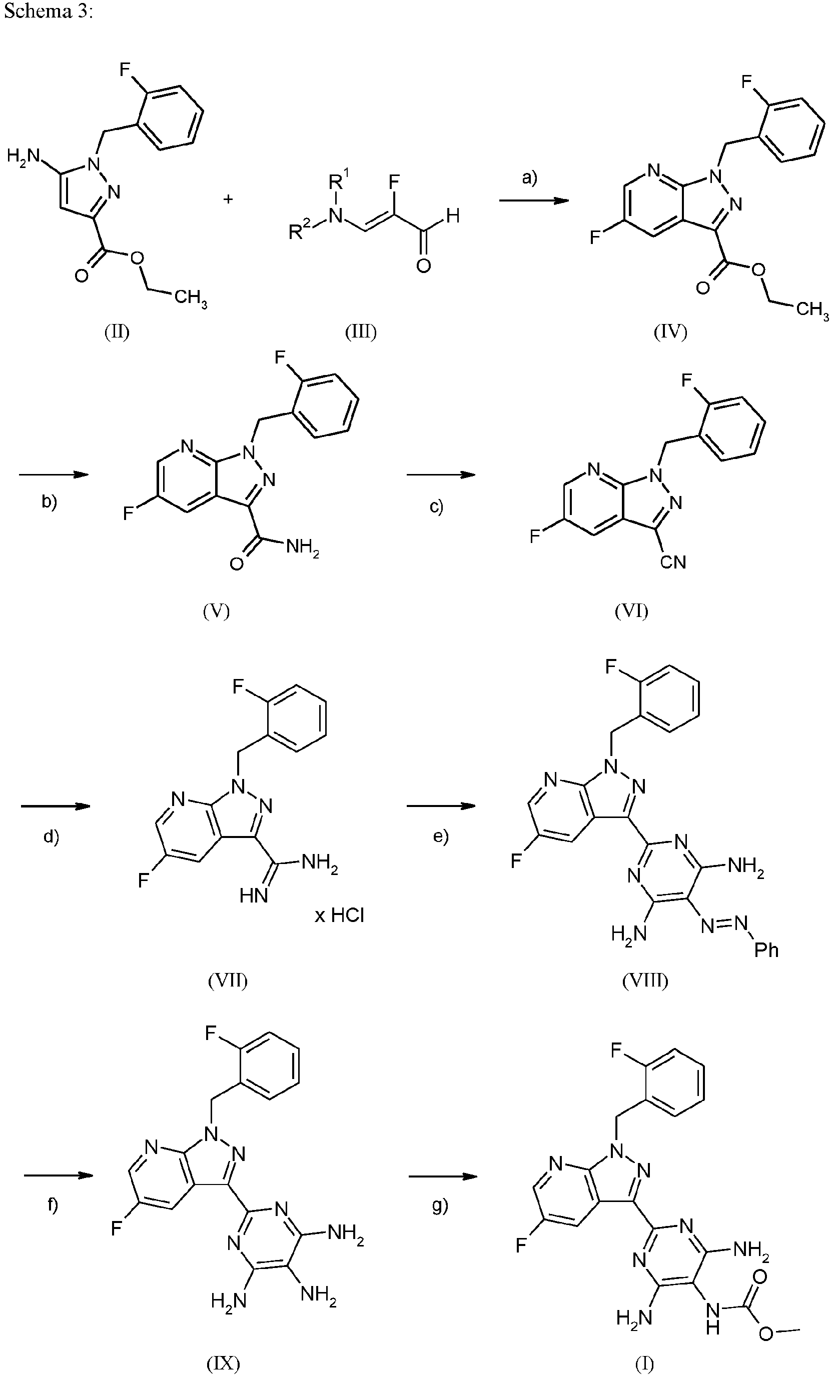

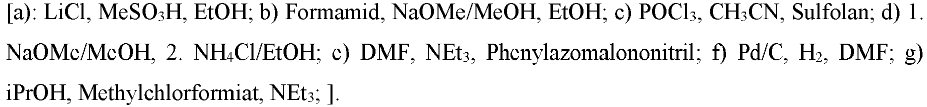

Der Schritt a) ist bereits für die unsubstituierten Pyrazolopyridine durch (WO 03/004503 (Beispiel IIIb) und WO 03/095451 (Beispiel 2A)) bekannt:

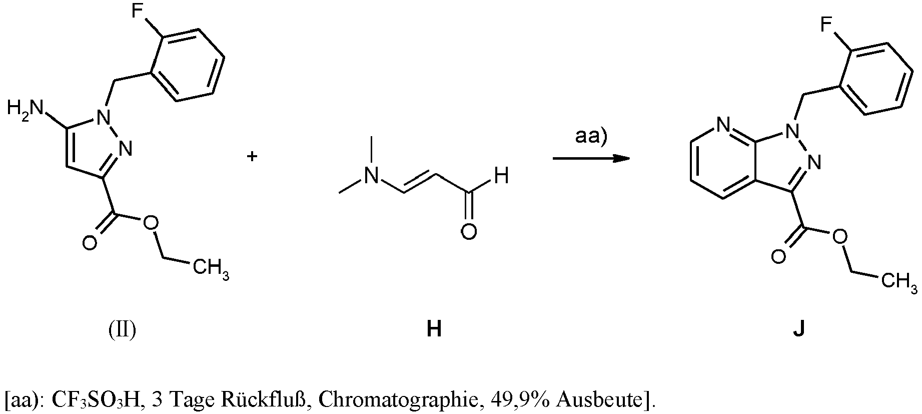

Im Gegensatz zum Stand der Technik (WO 03/004503, Beispiel IIIb und WO 03/095451, Beispiel 2A) verläuft die Herstellung von IV mit einer deutlich höheren Ausbeute.

Ein weiterer Vorteil ist, dass anstelle der korrosiven Trifluoressigsäure das viel preiswertere Ethanol als Lösungsmittel verwendet wird.

Ein weiterer Vorteil ist, dass die Reaktionszeit in Gegensatz zum Stand der Technik erheblich kürzer ist.

Ein weiterer Vorteil ist, dass die Herstellung von IV mit hoher Selektivität erfolgt und das Produkt in hoher Reinheit ohne nennenswerte Nebenproduktbildung gebildet wird und aufwändige Reinigungsprozeduren nicht erforderlich sind.

Ein weiterer Vorteil ist, dass IV durch Kristallisation in hoher Reinheit und Ausbeute erhalten wird.

Die Schritte d) - g) sind bereits für die unsubstituierten Pyrazolopyridine durch WO 03/095451, WO 2011/064156 und WO 2011/064171 bekannt und können analog verwendet werden.

Im Einzelnen umfasst das offenbarungsgemäße Verfahren zur Herstellung einer Verbindung der Formel (VI) 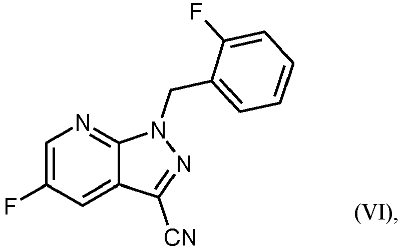 welches durch die Zyklisierung des 5-Aminopyrazol-Derivats (IIa) 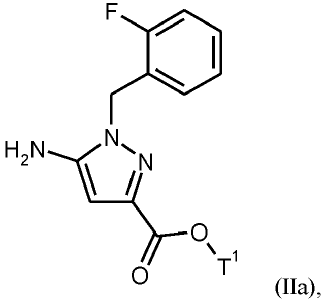 in welchem
- T¹: für (C₁-C₄)-Alkyl steht,
in Gegenwart einer geeigneten Säure mit dem Aldehyd (III) 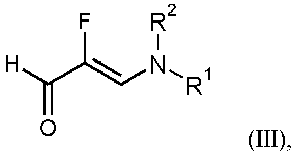 worin R¹ und R² unabhängig voneinander für Methyl, Ethyl, Isopropyl, Phenyl oder zusammen mit dem Stickstoffatom, an welches sie gebunden sind für 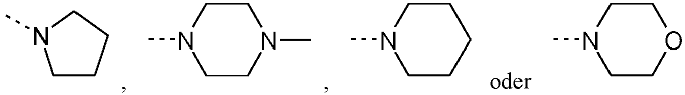 stehen, zum Ester der Formel (IVa) 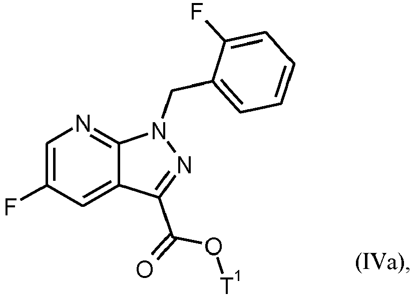 in welcher T¹ die zuvor angegebene Bedeutung hat,
dessen anschliessende Umsetzung mit Ammoniak oder Formamid zum Amid der Formel (V) 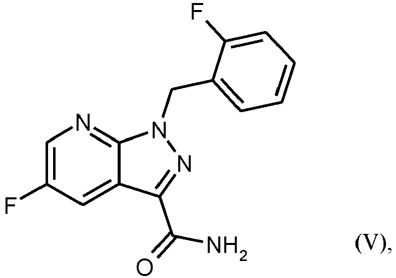 und die darauffolgende Dehydratisierung zum Nitril (VI).

Ein weiterer Gegenstand der vorliegenden Offenbarung ist die Verwendung der Verbindung der Formel (VI) 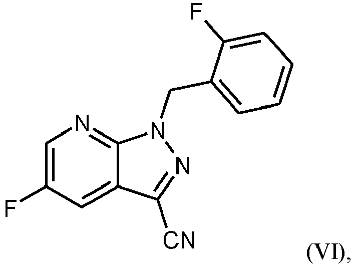 zur Herstellung der Verbindung der Formel (I) 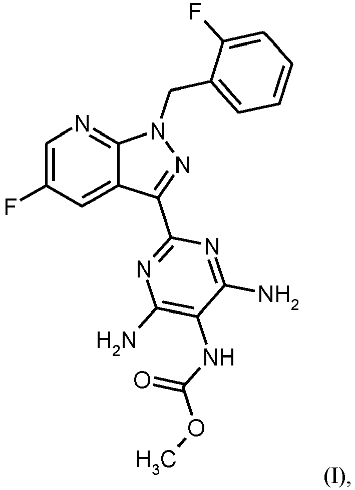 sowie deren *N*-Oxide, Salze, Solvate, Salze der *N*-Oxide und Solvate der *N*-Oxide und Salze.

Ein weiterer Gegenstand der vorliegenden Offenbarung ist die Verwendung der Verbindung der Formel (III) 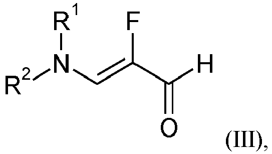 worin R¹ und R² unabhängig voneinander für Methyl, Ethyl, Isopropyl, Phenyl oder zusammen mit dem Stickstoffatom, an welches sie gebunden sind für 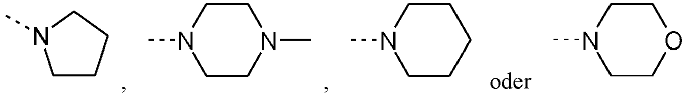 stehen, zur Herstellung der Verbindung der Formel (I) 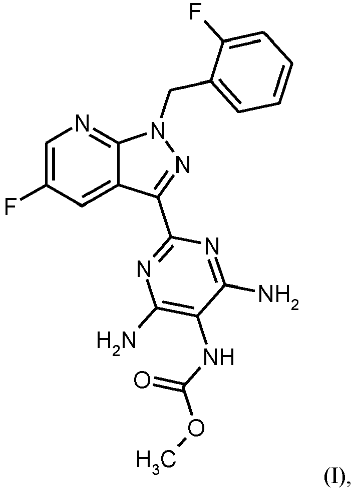 sowie deren *N*-Oxide, Salze, Solvate, Salze der *N*-Oxide und Solvate der *N*-Oxide und Salze.

Ein weiterer Gegenstand der vorliegenden Offenbarung ist die Verwendung der Verbindung der Formel (VI) zur Herstellung der Verbindung der Formel (I) wie oben angegeben, wobei die Verbindung der Formel (VI) in die Verbindung der Formel (VII) 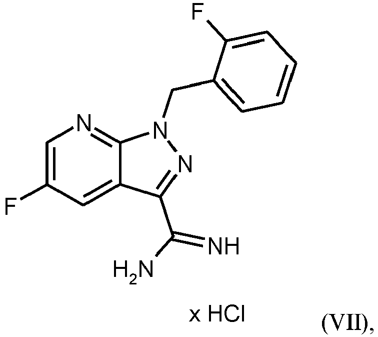 überführt, diese im Folgenden in einem inerten Lösungsmittel in Gegenwart einer geeigneten Base mit der Verbindung der Formel (VIIIa) 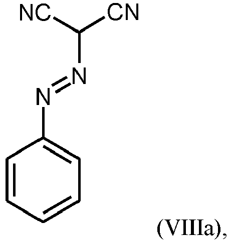 zur Verbindung der Formel (VIII) 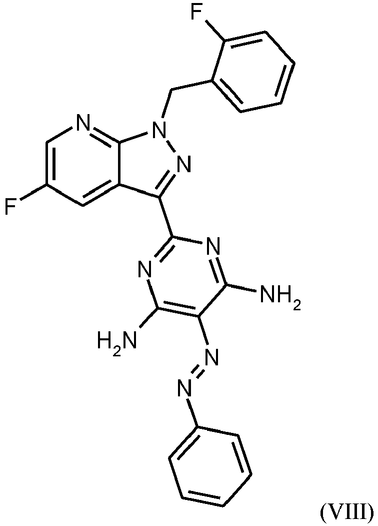 reagiert, und diese anschließend in einem inertem Lösungsmittel in Gegenwart eines geeigneten Reduktionsmittels zur Verbindung (IX) 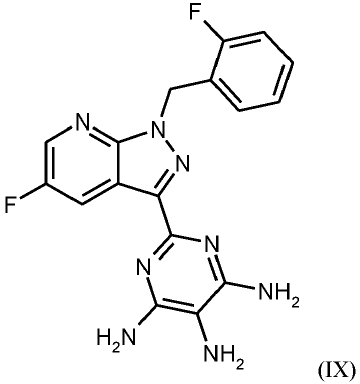 reduziert, diese im Anschluss in Gegenwart einer geeigneten Base mit oder ohne Lösungsmittel mit Chlorameisensäuremethylester oder mit Dimethyldicarbonat zur Verbindung der Formel (I) 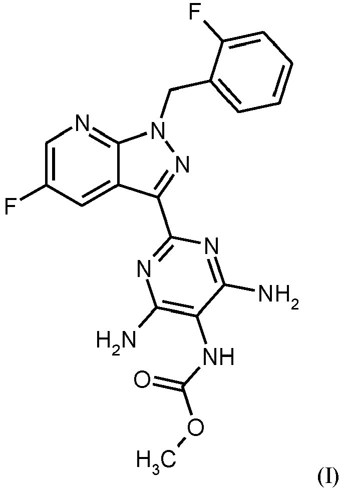 umsetzt,
und gegebenenfalls die resultierende Verbindung der Formel (I) gegebenenfalls mit den entsprechenden (i) Lösungsmitteln und/oder (ii) Säuren oder Basen in ihre Solvate, Salze und/oder Solvate der Salze überführt.

Die Umsetzung (VI) → (VII) erfolgt nach den dem Fachmann bekannten Methoden in einem zweistufigen Prozess zunächst unter Bildung des Iminoesters mit Natriummethanolat in Methanol bei 0°C bis +40°C und anschließender nucleophiler Addition eines Ammoniak-Äquivalents wie beispielsweise Ammoniak oder Ammoniumchlorid in Essigsäure oder einem Alkohol unter Bildung des Amidins (VII) bei +50 bis +150°C.

Geeignete Alkohole für die Umsetzung (VI) → (VII) sind Alkohole wie Methanol, Ethanol, n-Propanol, Isopropanol, n-Butanol oder tert.-Butanol.

Inerte Lösungsmittel für den Verfahrensschritt (VII) + (VIIIa) → (VIII) sind Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), Sulfolan, *N,N'*-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt sind DMF und Sulfolan.

Geeignete Basen für den Verfahrensschritt (VII) + (VIIIa) → (VIII) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Triethylamin.

Die Reaktion (VII) + (VIIIa) → (VIII) wird im Allgemeinen in einem Temperaturbereich von +20°C bis +150°C, bevorzugt bei +80°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Verbindung der Formel (VIIIa) kann analog Literatur L.F.Cavalieri, J.F.Tanker, A.Bendich, J.Am.Chem.Soc., 1949, 71, 533 dargestellt werden.

Die Reduktionen (VIII) → (IX) erfolgen in Gegenwart eines geeigneten Katalysators in einem inerten Lösungsmittel, in einem Temperaturbereich von +20°C bis +100°C unter Wasserstoff-druck (z.B. von 1 bis 100 bar). Bevorzugt ist ein Temperaturbereich von 40°C bis 80°C und ein Wasserstoffdruckbereich von 5 bis 70 bar.

Inerte Lösungsmittel für die Reduktion (VIII) → (IX) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt sind DMF und Pyridin.

Geeignete Katalysatoren für die Umsetzung (VIII) → (IX) sind beispielsweise Palladium auf Aktivkohle, Platin auf Kohle, Palladiumhydroxid oder Raney-Nickel.

Die Reduktion (VIII) → (IX) kann alternativ mit einem Metall bzw. Metallsalz wie beispielsweise Eisen, Zink oder Zinn(II)chlorid in einer geeigneten Säure wie beispielsweise Chlorwasserstoff/ Salzsäure, Schwefelsäure, Phosphorsäure oder Essigsäure in einem Temperaturbereich von +20°C bis +140°C erfolgen.

Inerte Lösungsmittel für den Verfahrensschritt (IX) → (I) sind beispielsweise Alkohole wie Methanol, Ethanol, n-Propanol, Isopropanol, n-Butanol oder tert.-Butanol, Ether wie Diethylether, Diisopropylether, Dioxan, Tetrahydrofuran, Glykoldimethylether oder Diethylenglykoldimethylether, Halogenkohlenwasserstoffe wie Dichlormethan, Trichlormethan, Tetrachlormethan, Trichlorethylen oder Chlorbenzol, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Acetonitril Ethylacetat oder auch Wasser. Ebenso ist es möglich, Gemische der genannten Lösungsmittel einzusetzen. Bevorzugt sind Isopropanol und Tetrahydrofuran sowie ein Gemisch aus Isopropanol und Tetrahydrofuran.

Geeignete Basen für den Verfahrensschritt (IX) → (I) sind Alkalihydride wie Natriumhydrid, Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 4-Dimethylaminopyridin, 1,8-Diazabicyclo[5.4.0]undec-7-en (DBU) oder 1,5-Diazabicyclo[4.3.0]non-5-en (DBN). Bevorzugt ist Triethylamin.

Die Reaktion (IX) → (I) wird im Allgemeinen in einem Temperaturbereich von -10°C bis +70°C, bevorzugt bei 0°C bis +50°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Verbindungen der Formel (IIa) sind literaturbekannt und können in Analogie zu Beispiel 20A in WO 00/06569 hergestellt werden.

Verbindungen der Formel (III) sind aus der Literatur H. Yamanaka, S. Yamashita and T. Ishihara, Synlett 353-354 (1993) bekannt. Die dort offenbarte Synthese wird in Schema 4 veranschaulicht.

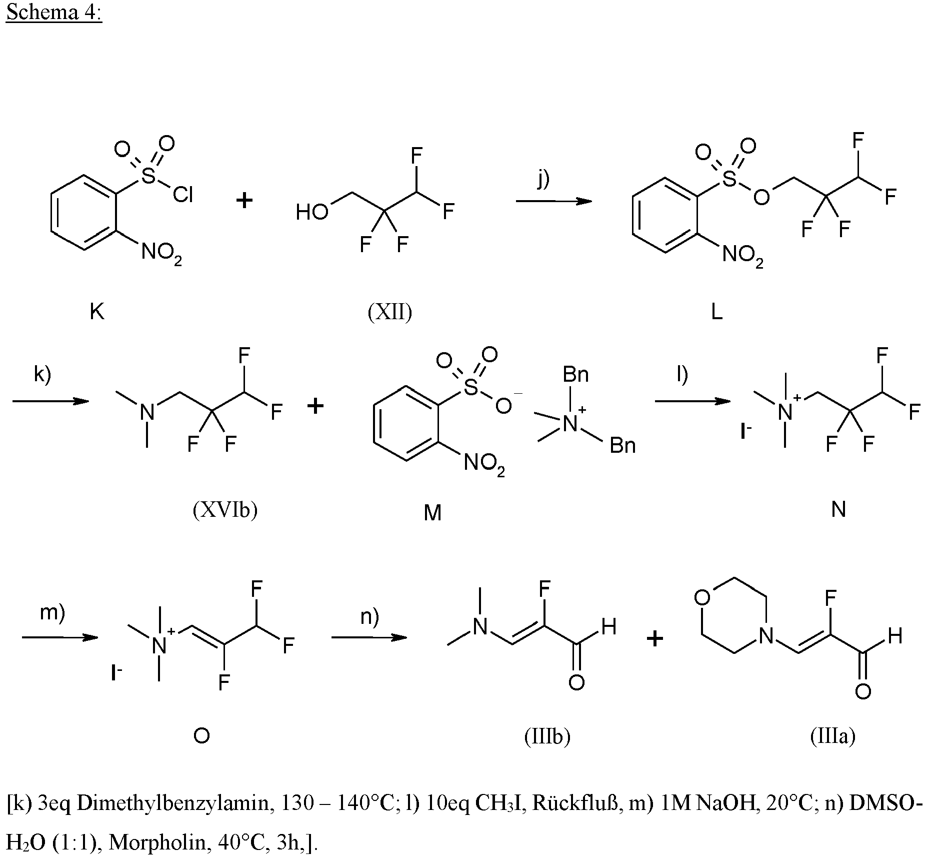

Nachteil dieses Verfahrens ist, dass bei der Herstellung von (XVIb) gemäß H. Yamanaka, M.Kuwabara, M. Okudo, K. Fukunishi and M. Nomura, Nippon Kagaku Kaishi (10) 1988-1994 (1985) nur eine Ausbeute von 66% erzielt wird und bei diesem Prozess sehr große Mengen (2,79kg pro kg (XVIb)) an Nebenprodukt (Dimethyldibenzyl nitrobenzolsulfonat) anfallen, die abgetrennt und entsorgt werden müssen.

Ein weiterer Nachteil dieses Verfahrens ist, dass gemäß H. Yamanaka, H. Ganbayashi, M.Kuwabara,K. Fukunishi and M. Nomura, Nippon Kagaku Kaishi (7) 1036-1043 (1988) ausgehend von (XVIb) die Alkylierung 10 Äquivalente des cancerogenen Alkylierungsmittels Methyliodid erfordert.

Ein weiterer Nachteil dieses Verfahrens ist, dass gemäß H. Yamanaka, S. Yamashita and T. Ishihara, Synlett 353-354 (1993) bei der Umsetzung von O mit Morpholin neben dem gewünschten Produkt (IIIb) auch 11% des Nebenprodukts (IIIa) entstehen was eine aufwändige Reinigung erforderlich macht und zur Folge hat, dass die Gesamtsynthese zur Herstellung von (IIIb) nur eine geringe Gesamtausbeute liefert und hohe Herstellungskosten verursacht.

Die dort beschriebene Synthese eignet sich jedoch nicht für die Herstellung der Aldehyde der Formel (III) im technischen Maßstab, sodass gemäß vorliegender Offenbarung eine neue und effiziente Synthese entwickelt wurde, die in Schema 5 beispielhaft veranschaulicht wird.

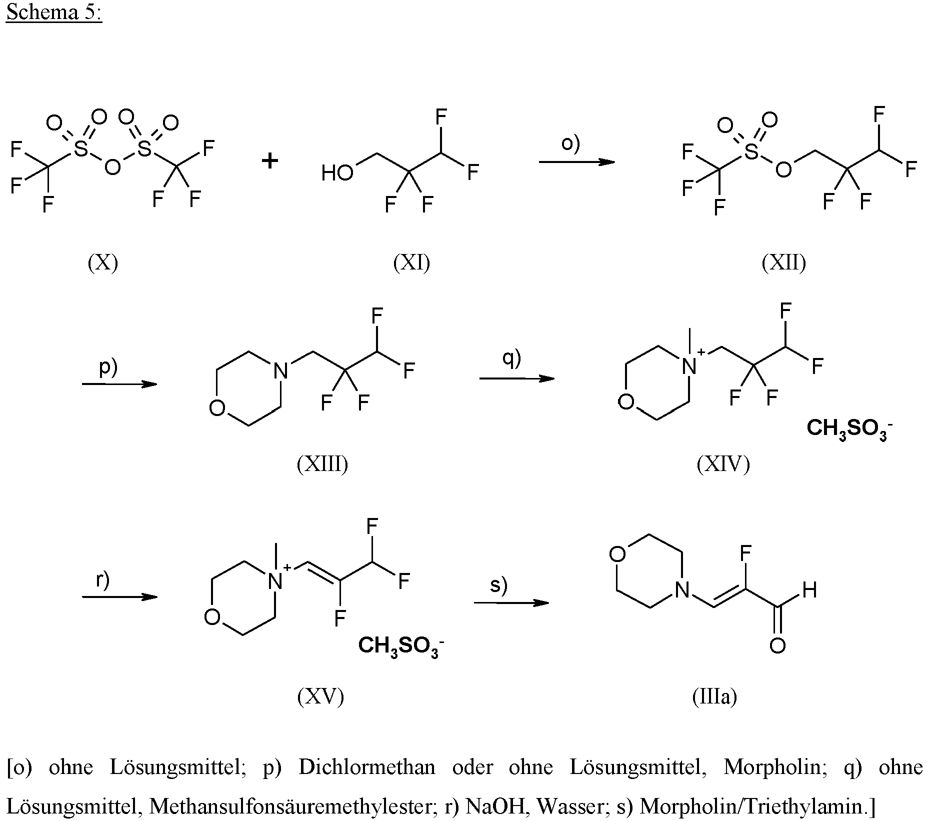

Die Verbindung der Formel (XIII) ist laut Literatur Markovskii, L. N.; Kolesnik, N. P.; Shermolovich, Yu. G Zhurnal Obshchei Khimii (1980), 50(4), 826-829 bekannt. Die dort offenbarte Synthese wird in Schema 6 veranschaulicht.

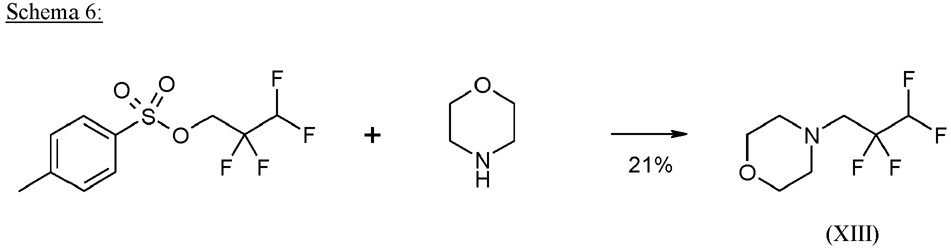

Die dort beschriebene Synthese eignet sich jedoch unter anderem wegen der geringen Ausbeute nicht für die Herstellung der Aldehyde der Formel (III) im technischen Maßstab.

Ein weiterer Gegenstand der vorliegenden Offenbarung ist ein Verfahren zur Herstellung von Verbindungen der Formel (III) 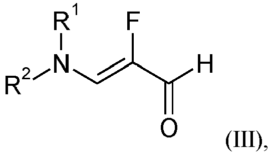 worin R¹ und R² unabhängig voneinander für Methyl, Ethyl, Isopropyl, Phenyl oder zusammen mit dem Stickstoffatom, an welches sie gebunden sind für 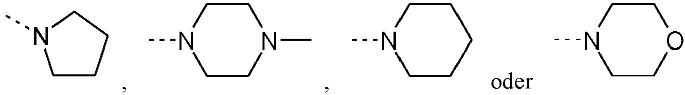 stehen, wobei Trifluormethansulfonsäureanhydrid der Formel (X) mit 2,2,3,3-Tetrafluoro-1-propanol der Formel (XI) ohne Lösungsmittel umgesetzt wird und das resultierende 2,2,3,3-Tetrafluorpropyltrifluormethansulfonat der Formel (XII) mit einer Verbindung der Formel (XIIa) 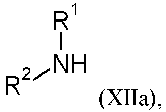
worin R¹ und R² die oben angegebenen Bedeutungen aufweisen,
   zu einer Verbindung der Formel (XIIIa) 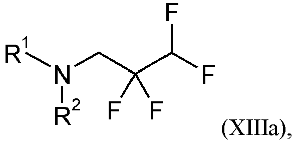
worin R¹ und R² die oben angegebenen Bedeutungen aufweisen,
   und mit Methansulfonsäuremethylester zu einer Verbindung der Formel (XIVa) 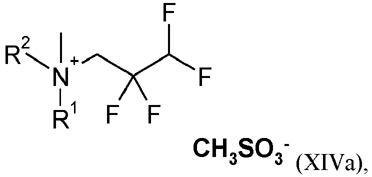
worin R¹ und R² die oben angegebenen Bedeutungen aufweisen,
   und mit Natriumhydroxid zu einer Verbindung der Formel (XVa) 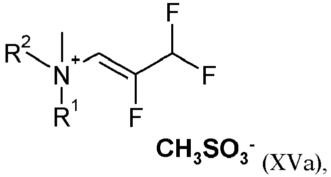
worin R¹ und R² die oben angegebenen Bedeutungen aufweisen,
   und abschliessend unter basischen Bedingungen zu der Verbindung der Formel (III) umgesetzt wird.

Ein weiterer bevorzugter Gegenstand der vorliegenden Offenbarung ist ein Verfahren zur Herstellung von Verbindungen der Formel (IIIa) 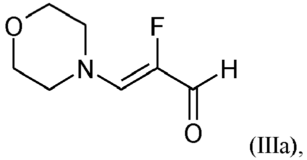 wobei Trifluormethansulfonsäureanhydrid der Formel (X) mit 2,2,3,3-Tetrafluoro-1-propanol der Formel (XI) ohne Lösungsmittel umgesetzt wird und das resultierende 2,2,3,3-Tetrafluorpropyltrifluormethansulfonat der Formel (XII) mit Morpholin zu einer Verbindung der Formel (XIII) 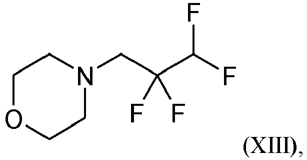 und mit Methansulfonsäuremethylester zu einer Verbindung der Formel (XIV) 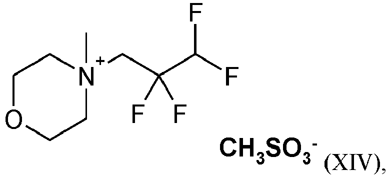 und mit Natriumhydroxid zu einer Verbindung der Formel (XV) 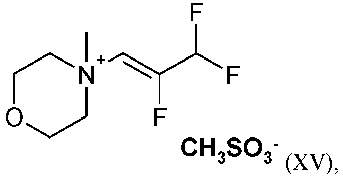 und abschließend unter Zugabe von Morpholin zu der Verbindung der Formel (III) umgesetzt wird.

Die neue Synthese hat gegenüber dem Stand der Technik den Vorteil, dass die Zwischenstufe (XII) sowie die bislang unbekannten Zwischenstufen (XIV) und (XV) nicht isoliert werden müssen, was den technischen Aufwand der Synthese stark reduziert.

Auch die Ausbeuten der resultierenden Aldehyde der Formel (III) sind mit dem neuen Syntheseverfahren deutlich höher als im Stand der Technik.

Basische Bedingungen im Sinne der Offenbarung bedeutet für den Verfahrensschritt (XIVa) zu (XVa) dass die bei der Reaktion gebildete Säure durch Hilfsbasen wie z.B. Natriumhydroxid, Kaliumhydroxid, Kaliumcarbonat, Natriumcarbonat, oder Triethylamin unter Bildung der entsprechenden Salze abgefangen wird.

Im Gegensatz zum Stand der Technik verläuft die Herstellung von (XIII) mit einer deutlich höheren Ausbeute. Vorteilhaft ist, dass zur Herstellung von (XII) kein Lösungsmittel erforderlich ist und dass die Zwischenstufe XII ohne weitere Reinigung in die Folgestufe zu (XIII) eingesetzt wird.

Ein weiterer Vorteil dieses Verfahrens ist, dass bei der Herstellung von (XIII) keine nennenswerten Abfälle gebildet werden. Vorteilhaft ist auch, dass aus dem gebildeten Morpholinium trifluormethansulfonat die Trifluormethansulfonsäure sowie Morpholin wiedergewonnen werden können.

Im Gegensatz zum Stand der Technik wird bei der Herstellung von (XIV) nur ein Äquivalent des Alkylierungsmittels benötigt. Die Reaktion wird ohne Lösungsmittel durchgeführt und verläuft praktisch quantitativ, wodurch eine hohe Raum-Zeitausbeute erzielt wird.

Ein weiterer Vorteil dieses Verfahrens ist, dass das Produkt (XIV) nicht isoliert wird, (XIV) wird in Wasser gelöst und diese Lösung mit Natronlauge zu (XV) umgesetzt.

Ein weiterer Vorteil dieses Verfahrens ist, dass auch das Produkt (XV) nicht isoliert wird, durch Umsetzung der wässrigen Lösung mit Morpholin wird (IIIa) als einziges Produkt in hoher Ausbeute erhalten.

Ein weiterer Vorteil dieses Verfahrens ist, dass (IIIa) durch Kristallisation in hoher Gesamtausbeute und Reinheit erhalten wird.

Die Zyklisierung des 5-Aminopyrazol-Derivats der Verbindung (IIa) mit dem Aldehyd der Verbindung (III) zur Verbindung der Formel (IV) erfolgt in einem inerten Lösungsmittel gegebenenfalls in Gegenwart einer Säure und ggf. eines Alkalimetallsalzes in einem Temperaturbereich von +10°C bis +200°C, bevorzugt bei +20°C bis +100°C, bei Normaldruck, innerhalb von beispielsweise 2 bis 50 Stunden, bevorzugt innerhalb von 2 bis 20 Stunden.

Säuren sind beispielsweise Salzsäure, Trifluoressigsäure und Methansulfonsäure. Bevorzugt sind Methansulfonsäure und Salzsäure.

Alkalimetallsalze sind Natriumchlorid oder Lithiumchlorid. Bevorzugtes Alkalimetallsalz ist Lithiumchlorid.

Inerte Lösungsmittel sind beispielsweise Alkohole wie Methanol, Ethanol, n-Propanol oder *iso*Propanol, n-Butanol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen oder andere Lösungsmittel, Acetonitril oder *N,N*-Dimethylformamid, oder Gemische von Lösungsmitteln. Bevorzugt sind Ethanol, Diethylenglykoldimethylether oder Dioxan.

Die bevorzugte Bildung des Amids (IVa) → (V) erfolgt durch Umsetzung in einem inertem Lösungsmittel mit Formamid in Gegenwart einer Base in einem Temperaturbereich von 0°C bis + 150°C, bevorzugt von +20°C bis +130°C, bei Normaldruck oder erhöhtem Druck, innerhalb von 2 bis 24 Stunden.

Inerte Lösungsmittel sind beispielsweise Alkohole wie Methanol, Ethanol, n-Propanol oder *iso*Propanol. Bevorzugt ist Ethanol.

Geeignete Basen für den bevorzugten Verfahrensschritt (IVa) → (V) sind Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]-non-5-en (DBN). Bevorzugt sind Natriummethanolat und Natriumethanolat,

Die Bildung des Amids (IVa) → (V) erfolgt alternativ durch Umsetzung mit Ammoniak in einem Temperaturbereich von 0°C bis + 50°C, bevorzugt von +20°C bis +30°C, bei Normaldruck oder erhöhtem Druck, innerhalb von 24 bis 72 Stunden.

Inerte Lösungsmittel sind beispielsweise Alkohole wie Methanol, Ethanol, n-Propanol oder *iso*Propanol. Bevorzugt wird eine Lösung von Ammoniak in Methanol in einer Konzentration von 5N bis 7N eingesetzt.

Die Dehydratisierung des Amid (V) zum Nitril (VI) erfolgt in einem inerten Lösungsmittel gegebenenfalls in Gegenwart einer geeigneten Base mit einem geeigneten Dehydatisierungsmittel wie beispielsweise Phosphoroxychlorid, Trifluoressigsäureanhydrid, Essigsäureanhydrid oder Trifluormethansulfonsäureanhydrid, in einem Temperaturbereich von 0°C bis +150°C, bevorzugt bei +50°C bis +110°C, innerhalb von 1 bis 12 Stunden.

Bevorzugt ist Phosphoroxychlorid.

Inerte Lösungsmittel sind Ether wie Diethylether, Dioxan, Tetrahydrofuran (THF), Glykoldimethylether oder Diethylenglykoldimethylether, Kohlenwasserstoffe wie Benzol, Toluol, Xylol, Hexan, Cyclohexan oder Erdölfraktionen oder andere Lösungsmittel, Pyridin, Sulfolan, Acetonitril oder *N,N*-Dimethylformamid, oder Gemische von Lösungsmitteln. Bevorzugt ist Sulfolan und Acetonitril.

Geeignete Basen sind beispielsweise organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Pyridin.

Die im Rahmen des Verfahrens gemäß der Offenbarung beschriebenen Verbindungen können auch in Form ihrer Salze, Solvate oder Solvate der Salze vorliegen.

Die im Rahmen des Verfahrens gemäß der Offenbarung beschriebenen Verbindungen können in Abhängigkeit von der Struktur auch in Form ihrer Tautomere vorliegen.

Als Salze sind im Rahmen des offenbarten Verfahrens physiologisch unbedenkliche Salze der im Verfahren gemäß der Offenbarung eingesetzten und hergestellten Verbindungen bevorzugt.

Physiologisch unbedenkliche Salze der im Verfahren gemäß der Offenbarung eingesetzten und hergestellten Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der im Verfahren gemäß Offenbarung eingesetzten und hergestellten Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dihydroabiethylamin, Arginin, Lysin, Ethylendiamin und Methylpiperidin.

Als Solvate werden im Rahmen der Offenbarung solche Formen der im Verfahren gemäß Offenbarung eingesetzten und hergestellten Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Offenbarung haben die Substituenten, soweit nicht anders spezifiziert, die folgende Bedeutung:

Alkyl steht im Rahmen der Offenbarung für einen linearen oder verzweigten Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec.*-Butyl und *tert.-Butyl.*

Die vorliegende Offenbarung wird nachstehend anhand von nicht einschränkenden bevorzugten Beispielen und Vergleichsbeispielen näher dargestellt. Soweit nicht anderweitig angegeben, beziehen sich alle Mengenangaben auf Gewichtsprozente.

Gegenstand der vorliegenden Offenbarung ist ein Verfahren zur Herstellung von Verbindungen der Formel (VI) 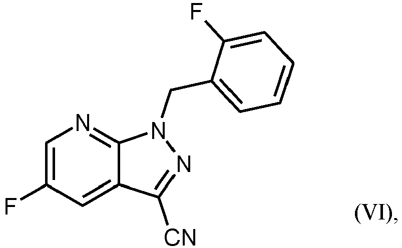 dadurch gekennzeichnet, dass die Verbindung der Formel (V) 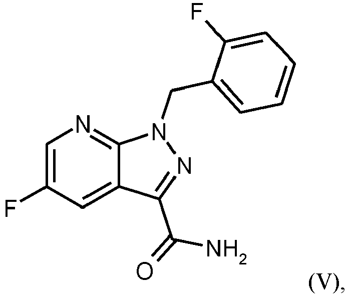 durch Umsetzung eines Esters der Formel (IVa) 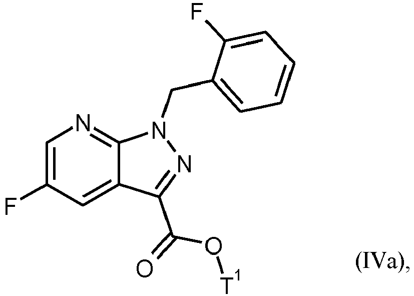 in welcher
- T¹: für (C₁-C₄)-Alkyl steht,
mit Formamid hergestellt wird.

Ein weiterer Gegenstand der vorliegenden Offenbarung ist ein Verfahren wie oben dargestellt, dadurch gekennzeichnet, dass ein Ester der Formel (IVa) durch Zyklisierung des 5-Aminopyrazol-Derivats (IIa) 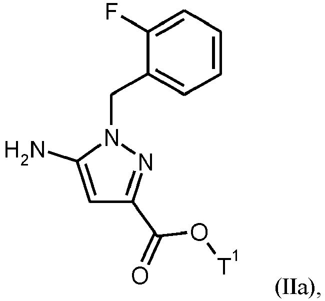 in welcher
- T¹: für (C₁-C₄)-Alkyl steht,
in Gegenwart einer Säure und eines Alkalimetallsalzes mit einem Aldehyd der Formel (III) 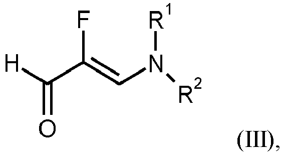 worin R¹ und R² unabhängig voneinander für Methyl, Ethyl, Isopropyl, Phenyl oder zusammen mit dem Stickstoffatom, an welches sie gebunden sind für 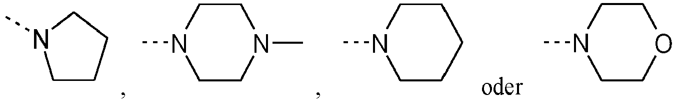 stehen, hergestellt wird.

Ein weiterer Gegenstand der vorliegenden Offenbarung ist ein Verfahren wie oben dargestellt, dadurch gekennzeichnet, dass als Aldehyd die Verbindung der Formel (IIIa) 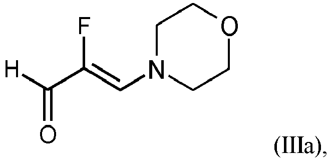 in der Zyklisierungsreaktion eingesetzt wird.

Ein weiterer Gegenstand der vorliegenden Offenbarung ist ein Verfahren zur Herstellung von Aldehyden der Formel (III) 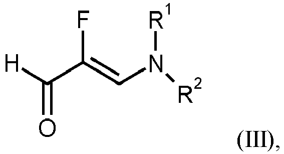 worin R¹ und R² unabhängig voneinander für Methyl, Ethyl, Isopropyl, Phenyl oder zusammen mit dem Stickstoffatom, an welches sie gebunden sind für 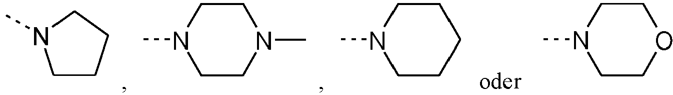 stehen, dadurch gekennzeichnet, dass Trifluormethansulfonsäureanhydrid mit 2,2,3,3-Tetrafluoro-1-propanol ohne Lösungsmittel umgesetzt wird und das resultierende 2,2,3,3-Tetrafluorpropyltrifluormethansulfonat mit einer Verbindung der Formel (XIIa) 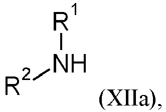 worin R¹ und R² die oben angegebenen Bedeutungen aufweisen, zu einer Verbindung der Formel (XIIIa) 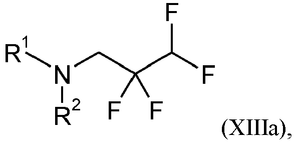 worin R¹ und R² die oben angegebenen Bedeutungen aufweisen,
und mit Methansulfonsäuremethylester zu einer Verbindung der Formel (XIVa) 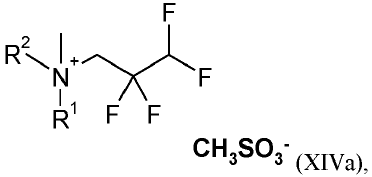 worin R¹ und R² die oben angegebenen Bedeutungen aufweisen,
und mit Natriumhydroxid zu einer Verbindung der Formel (XVa) 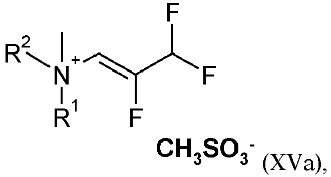 worin R¹ und R² die oben angegebenen Bedeutungen aufweisen,
und abschliessend unter basischen Bedingungen zu der Verbindung der Formel (III) umgesetzt wird.

Ein weiterer Gegenstand der vorliegenden Offenbarung ist ein Verfahren zur Herstellung von Verbindungen der Formel (IIIa) 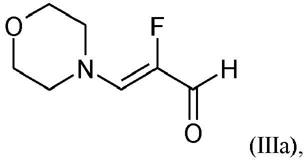 wobei Trifluormethansulfonsäureanhydrid der Formel (X) mit 2,2,3,3-Tetrafluoro-1-propanol der Formel (XI) ohne Lösungsmittel umgesetzt wird und das resultierende 2,2,3,3-Tetrafluorpropyltrifluormethansulfonat der Formel (XII) mit Morpholin zu einer Verbindung der Formel (XIII) 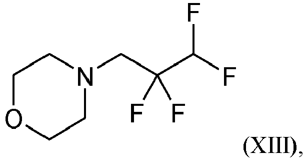 und mit Methansulfonsäuremethylester zu einer Verbindung der Formel (XIV) 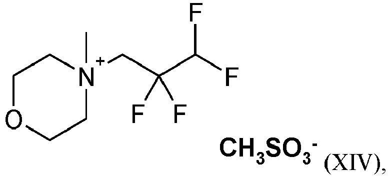 und mit Natriumhydroxid zu einer Verbindung der Formel (XV) 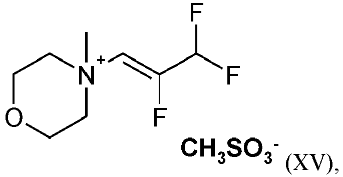 und abschliessend unter Zugabe von Morpholin zu der Verbindung der Formel (IIIa) umgesetzt wird.

Ein weiterer Gegenstand der vorliegenden Offenbarung ist ein Verfahren zur Herstellung von Verbindung der Formel (I) 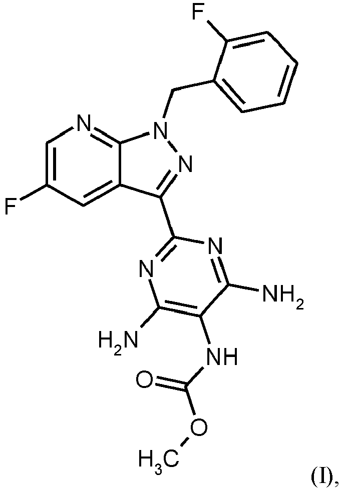 dadurch gekennzeichnet, dass Verbindungen der Formel (VI) 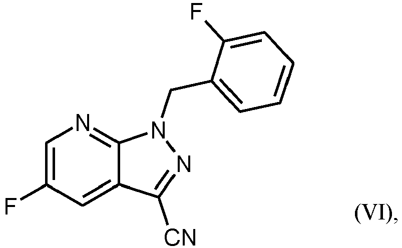 verwendet werden,
die dadurch gekennzeichnet sind, dass sie gemäß dem oben angegebenen Verfahren hergestellt werden und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Ein weiterer Gegenstand der vorliegenden Offenbarung ist ein Verfahren zur Herstellung von Verbindung der Formel (I) dadurch gekennzeichnet, dass Verbindungen der Formel (VI) 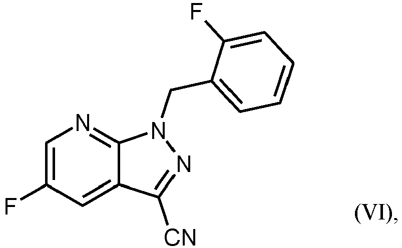 verwendet werden,
die dadurch gekennzeichnet sind, dass sie gemäß den oben angegebenen Verfahren hergestellt werden und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Ein weiterer Gegenstand der vorliegenden Offenbarung ist ein Verfahren zur Herstellung von Verbindung der Formel (I), dadurch gekennzeichnet, dass Verbindungen der Formel (VI) 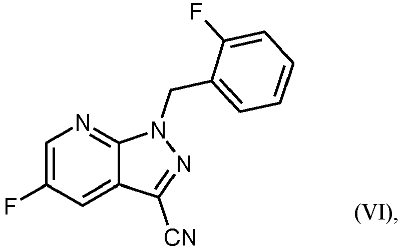 verwendet werden,
die dadurch gekennzeichnet sind, dass sie gemäß den oben angegebenen Verfahren hergestellt werden und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Ein weiterer Gegenstand der vorliegenden Offenbarung ist ein Verfahren zur Herstellung der Verbindung (I), dadurch gekennzeichnet, dass die Verbindung der Formel (VI) verwendet wird, die gemäß den oben angegebenen Verfahren hergestellt wird, wobei die Verbindung der Formel (VI) in die Verbindung der Formel (VII) 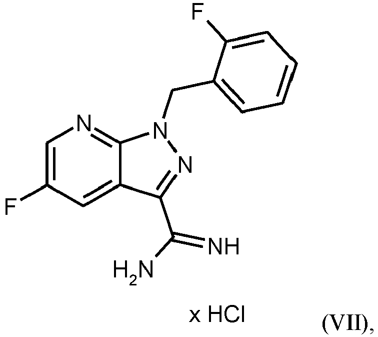 überführt, diese im Folgenden in einem inerten Lösungsmittel in Gegenwart einer geeigneten Base mit der Verbindung der Formel (VIIIa) 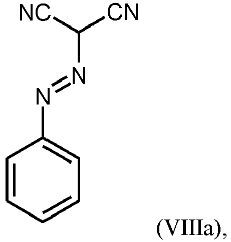 zur Verbindung der Formel (VIII) 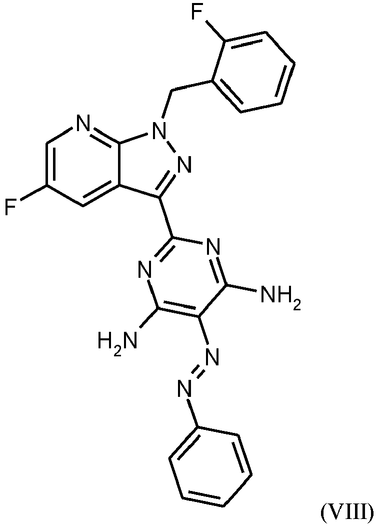 reagiert, und diese anschließend in einem inertem Lösungsmittel in Gegenwart eines geeigneten Reduktionsmittels zur Verbindung (IX) 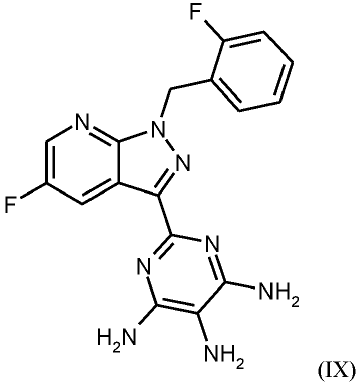 reduziert wird und die im Anschluss in Gegenwart einer geeigneten Base mit oder ohne Lösungsmittel mit Chlorameisensäuremethylester oder mit Dimethyldicarbonat zur Verbindung der Formel (I) umgesetzt wird, und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Ein Gegenstand der vorliegenden Erfindung ist die Verbindung der Formel (I) in kristalliner Form der Modifikation I 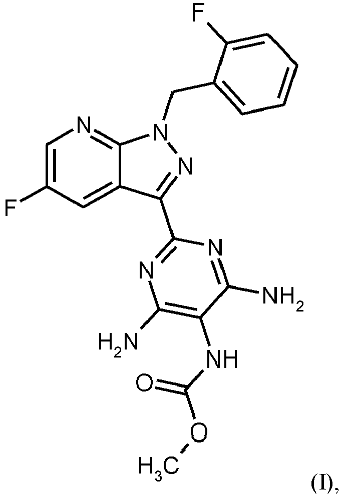 dadurch gekennzeichnet, dass das Röntgendiffraktogramm der Verbindung Peakmaxima des 2 Theta Winkels bei 5.9, 6.9, 16.2, 16.5, 24.1, 22.7, 24.7 zeigt. Ein weiterer Gegenstand der vorliegenden Erfindung ist die Verbindung der Formel (I) in kristalliner Form der Modifikation I 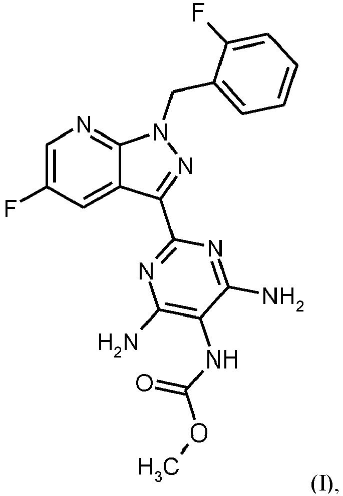 dadurch gekennzeichnet, dass das IR-Spektrum der Verbindung Bandenmaxima bei 1707, 1633, 1475 cm⁻¹ zeigt.

Verbindung der Formel (I) in der Modifikation (I) wie oben beschrieben, dadurch gekennzeichnet, dass das IR-Spektrum der Verbindung Bandenmaxima bei 1707, 1633, 1566, 1475, 1255, 1223 cm⁻¹ zeigt.

Ein weiterer Gegenstand der vorliegenden Erfindung ist ein Verfahren zur Herstellung der Verbindung der Formel (I) in kristalliner Form der Modifikation I, dadurch gekennzeichnet, dass die Verbindung der Formel (I) vorliegend als Di-Dimethylsulfoxid-Solvat in einem inerten Lösungsmittel bei einer Temperatur von 20°C - 120°C gerührt und die Verbindung der Formel (I) in der kristallinen Modifikation I isoliert wird.

Bevorzugte Lösungsmittel für das Verfahren zur Herstellung der Verbindung der Formel (I) in kristalliner Form der Modifikation I sind ein Gemisch aus Ethylacetat/ Ethanol/Wasser, Isopropanol, ein Gemisch aus Isopropanol/Wasser, Methanol, ein Gemisch aus Methanol/Wasser, Acetonitril, Aceton, Tetrahydrofuran und Methyl-tert.-butylether.

Ein bevorzugter Temperaturbereich für das Verfahren zur Herstellung der Verbindung der Formel (I) in kristalliner Form der Modifikation I ist von 20°C bis 90°C.

Ein weiterer Gegenstand der vorliegenden Erfindung ist eine Verbindung der Formel (I) in der Modifikation (I) wie oben beschrieben zur Behandlung von Krankheiten.

Ein weiterer Gegenstand der vorliegenden Erfindung ist ein Arzneimittel enthaltend eine Verbindung der Formel (I) in der Modifikation (I) wie oben beschrieben und keine größeren Anteile einer anderen Form der Verbindung der Formel (I) in der Modifikation (I) wie oben beschrieben. Arzneimittel enthaltend eine Verbindung der Formel (I) in der Modifikation (I) wie oben beschrieben in mehr als 90 Gewichtsprozente bezogen auf die Gesamtmenge der enthaltenen Verbindung der Formel (I) in der Modifikation (I) wie oben beschrieben.

Ein weiterer Gegenstand der vorliegenden Erfindung ist die Verwendung der Verbindung der Formel (I) in der Modifikation (I) wie oben beschrieben. Zur Herstellung eines Arzneimittels zur Behandlung von Herz-Kreislauferkrankungen.

Ein weiterer Gegenstand der vorliegenden Erfindung ist die Methode zur Behandlung von Herz-Kreislauferkrankungen durch Verabreichung einer wirksamen Menge einer Verbindung der Formel (I) in der Modifikation (I) wie oben beschrieben.

Ein weiterer Gegenstand der vorliegenden Erfindung ist die Verbindung der Formel (I) als Di-Dimethylsulfoxid-Solvat 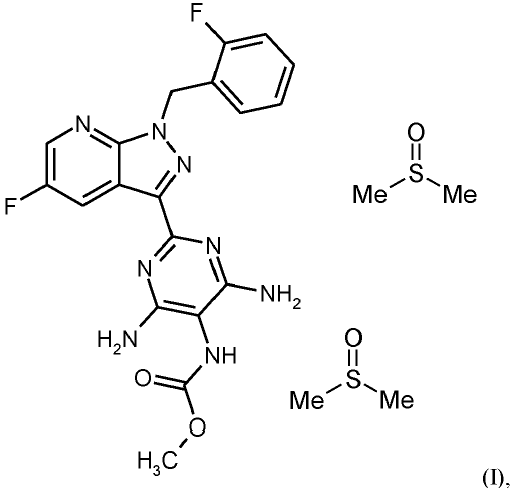 dadurch gekennzeichnet, dass das Röntgendiffraktogramm der Verbindung Peakmaxima des 2 Theta Winkels bei 12.0, 16.6, 17.8, 18.8, 20.3, 21.7 zeigt. Ein weiterer Gegenstand der vorliegenden Erfindung ist die Verbindung der Formel (I) als Di-Dimethylsulfoxid-Solvat 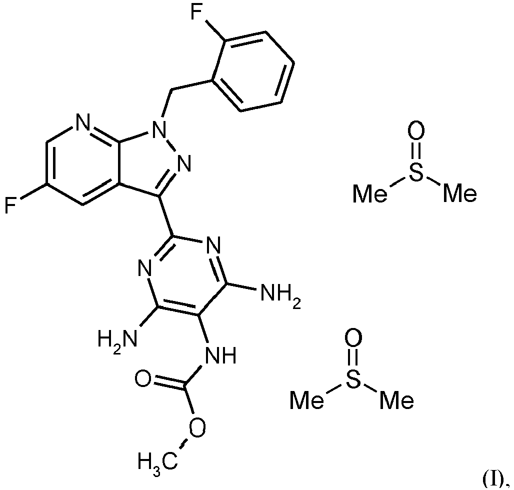 dadurch gekennzeichnet, dass das IR-Spektrum der Verbindung Bandenmaxima bei 1720, 1628, 1481 cm⁻¹ zeigt.

Ein weiterer Gegenstand der vorliegenden Erfindung ist die Verbindung der Formel (I) als Di-Dimethylsulfoxid-Solvat, dadurch gekennzeichnet, dass das IR-Spektrum der Verbindung Bandenmaxima bei 1720, 1628, 1481, 1234, 1041, 1017 cm⁻¹ zeigt.

Ein weiterer Gegenstand der vorliegenden Offenbarung ist ein Verfahren zur Herstellung der Verbindung der Formel (I) als Di-Dimethylsufoxid-Solvat in kristalliner Form, dadurch gekennzeichnet, dass die Verbindung der Formel (I) vorliegend in einer oder mehrere Modifikationen oder als Solvat in Dimethylsulfoxid oder einen Gemisch aus Dimethylsulfoxid und einem inerten Lösungsmittel, wie bspw. Ethylacetat bei einer Temperatur von 20 - 120°C gerührt und das Di-Dimethylsulfoxid-Solvat isoliert wird. Bevorzugt ist ein Temperaturbereich von 20 bis 90°C.

Ein weiterer Gegenstand der vorliegenden Offenbarung ist die Verbindung der Formel (XIV) 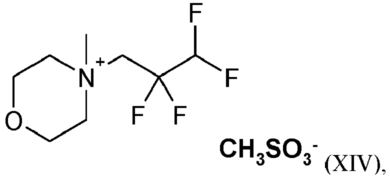 sowie ihre Salze, Solvate und Solvate der Salze.

Ein weiterer Gegenstand der vorliegenden Offenbarung ist die Verbindung der Formel (XV) 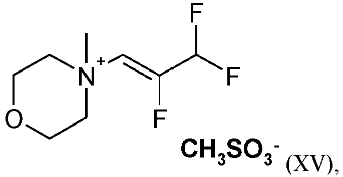 sowie ihre Salze, Solvate und Solvate der Salze.

### A. Beispiele

### Abkürzungen:

- Ac: Acetyl
- CI: chemische Ionisation (bei MS)
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- GC/MS: Gaschromatographie-gekoppelte Massenspektrometrie
- ges.: Gesättigt
- H: Stunde(n)
- HPLC: Hochdruck-, Hochleistungsflüssigchromatographie
- HV: Hochvakuum
- konz.: Konzentriert
- LC/MS: Flüssigchromatographie-gekoppelte Massenspektrometrie
- Me: Methyl
- Min: Minute(n)
- MS: Massenspektrometrie
- NMR: Kernresonanzspektrometrie
- Rac: racemisch / Racemat
- R_{f}: Retentionsfaktor (bei Dünnschichtchromatographie auf Kieselgel)
- RT: Raumtemperatur
- Rₜ: Retentionszeit (bei HPLC)
- SFC: Supercritical Fluid Chromatography
- THF: Tetrahydrofuran
- UV: Ultraviolett-Spektrometrie
- v/v: Volumen zu Volumen-Verhältnis (einer Lösung)

### Alle Röntgenpulverdiffraktometrie-Daten wurden mit folgenden Akquisitionsparametern erhalten:

- Diffractometer system: PANalytical XPERT-PRO
- Scan-Achse: Gonio
- Anodenmaterial: Cu
- K-Alphal [Å]: 1,54060
- K-Alpha2 [Å]: 1,54443
- K-A2 / K-A1 Verhältnis: 0,50000
- Scan Modus:: Transmission
- Scan Typ:: 2Theta:Omega
- Angabe 2Theta:: ± 0,2°

### Alle Infrarotspektroskopie-Daten wurden mit den folgenden Aquisitionsparamentern erhalten:

- Spektrometer:: Perkin Elmer Spectrum One mit Diamant ATR-Einheit
- Parameter:: 32 Scans
- Auflösung:: 2 cm⁻¹

### Gegenstand der Offenbarung ist Beispiel 1

### 2,2,3,3-Tetrafluorpropyltrifluormethansulfonat

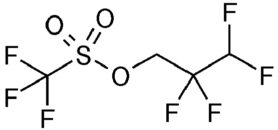

### Methode A:

252,5 g (0,895 mol) Trifluormethansulfonsäureanhydrid wurden auf 40°C erhitzt und bei dieser Temperatur unter Kühlung 130,0 g (0,984 mol) 2,2,3,3-Tetrafluoro-1-propanol zudosiert. Nach Beendigung der Dosierung wurde das Reaktionsgemisch auf 70°-75°C erhitzt und 2h gerührt. Es wurde auf 20°C gekühlt und die Reaktionslösung ohne weitere Aufreinigung in die Reaktion zu Beispiel 2 eingesetzt.

### Methode B:

50,0 g (0,379 mol) 2,2,3,3-Tetrafluoro-1-propanol wurden auf 0°C gekühlt und bei 0° - 4°C 106,8 g (0,379 mol) Trifluormethansulfonsäureanhydrid zugetropft. Anschließend wurde das Reaktionsgemisch 2h bei 25°C gerührt, auf 70°-75°C erhitzt und 2h gerührt. Es wurde auf 20°C gekühlt und die Reaktionslösung bei 116° - 118°C destilliert. Man erhielt 85,1 g (85,1 % d. Th.) der Titelverbindung.
¹H-NMR (400 MHz, CDCl₃): δ = 4.69 (t, *J*=11.86 Hz, 2 H) 5.54 - 6.23 (m, 1 H) ppm.

### Gegenstand der Offenbarung ist Beispiel 2

### 4-(2,2,3,3-Tetrafluorpropyl)morpholin

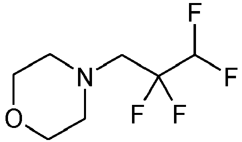

### Methode A:

311,9 g (3,58 mol) Morpholin wurden in 290 ml Dichlormethan gelöst und auf -15°C gekühlt. Bei - 15° - 0°C wurden 371,4g (max. 0,895 mol) der Reaktionslösung aus Beispiel 1 unter Kühlung zugetropft und anschließend 30 min bei 0° - 5°C gerührt. Das Reaktionsgemisch wurde auf 40°C erhitzt und 4,5h gerührt. Nach Abkühlen auf 20°C wurden 320 ml Wasser zugegeben und die Phasen getrennt. Die organische Phase wurde dreimal mit je 190 ml Wasser gewaschen und am Rotationsverdampfer bei 30°C/30mbar eingeengt. Der Rückstand (160,7 g) wurde bei 67° - 68°C/18 mbar destilliert. Man erhielt 151,7 g (84,3 % d. Th.) der Titelverbindung.
¹H-NMR (400 MHz, CDCl₃): δ = 2.53 - 2.70 (m, 4 H) 2.89 (tt, *J*=14.03, 1.74 Hz, 2 H) 3.61 - 3.78 (m, 4 H) 5.83 - 6.22 (m, 1 H) ppm.

### Methode B:

158,5 g (1,82 mol) Morpholin wurden auf 5°C gekühlt. Bei 5° - 10°C wurden 189,5g (max. 0,455 mol) der Reaktionslösung aus Beispiel 1 unter Kühlung zugetropft und anschließend 30 min bei 5° - 10°C gerührt. Das Reaktionsgemisch wurde auf 40°C erhitzt und 1h gerührt. Nach Abkühlen auf 20°C wurden 160 ml Wasser und 160 ml Toluol zugegeben und die Phasen getrennt. Die organische Phase wurde mit 160 ml Wasser gewaschen und am Rotationsverdampfer bei 50°C/50mbar eingeengt. Der Rückstand (81,0 g) wurde bei 67° - 68°C/18 mbar destilliert. Man erhielt 77,0 g (84,1 % d. Th.) der Titelverbindung.

### Gegenstand der Offenbarung ist Beispiel 3

### 4-Methyl-4-(2,2,3,3-tetrafluorpropyl)morpholin-4-ium methansulfonat

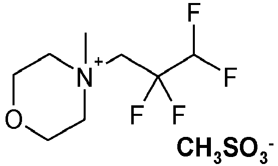

### Methode A:

143,7 g (1,31 mol) Methansulfonsäuremethylester wurden auf 135°C erhitzt und bei dieser Temperatur 250,0g (1,243 mol) der Verbindung aus Beispiel 2 zugetropft. Anschließend wurde 22 h bei 100°C gerührt. Das Reaktionsgemisch wurde auf 85°C abgekühlt und 375 ml Isopropanol zugegeben. Nach Abkühlen auf 0° - 5°C wurde 30 min nachgerührt und das Produkt abgesaugt. Es wurde dreimal mit je 125 ml Isopropanol gewaschen und im Vakuumtrockenschrank bei 45°C unter leichtem Stickstoffstrom getrocknet. Man erhielt 336,8 g (87,1 % d. Th.) der Titelverbindung.
¹H-NMR (400 MHz, D₂O): δ = 2.81 (s, 3 H) 3.55 (s, 3 H) 3.68 - 3.93 (m, 4 H) 4.01 - 4.24 (m, 4 H) 4.33 - 4.51 (m, 2 H) 6.13 - 6.48 (m, 1 H) ppm.

### Methode B:

20,0 g (181,3 mmol) Methansulfonsäuremethylester wurden auf 135°C erhitzt und bei dieser Temperatur 35,1g (172,7 mmol) der Verbindung aus Beispiel 2 zugetropft. Es wurde 3 h bei 135°C gerührt und anschließend 40 ml Wasser zugegeben. Nach Abkühlen auf 50°C wurde die wässrige Lösung der Titelverbindung in die Folgestufe (siehe Beispiel 4) eingesetzt.

### Gegenstand der Offenbarung ist Beispiel 4

### 4-Methyl-4-[2,3,3-trifluorprop-1-en-1-yl]morpholin-4-ium methansulfonat

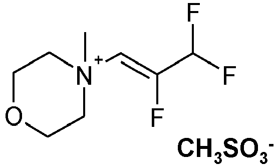

Zur wässrigen Lösung der Verbindung aus Beispiel 3, Methode B (max. 172,7 mmol) wurden bei 50° - 55°C 16,9g (189,9 mmol) Natronlauge 45%ig zudosiert und 1h bei 50°C gerührt. Das Reaktionsgemisch wurde auf 20°C gekühlt und die ausgefallenen Salze abgesaugt und mit 5 ml Wasser gewaschen. Die wässrige Produktlösung (102,1 g; max. 172,7 mmol) wurde in die Folgestufe (siehe Beispiel 5) eingesetzt.

Für analytische Zwecke wurde eine Probe eingeengt und getrocknet.
¹H-NMR (400 MHz, D₂O): δ = 2.81 (s, 3 H) 3.59 (s, 3 H) 3.76 - 3.85 (m, 2 H) 3.97 - 4.09 (m, 4 H) 4.12 - 4.20 (m, 2 H) 6.39 - 6.69 (m, 1 H) 6.74 - 6.83 (m, 1 H) ppm.

### Gegenstand der Offenbarung ist Beispiel 5

### 2-Fluor-3-(morpholin-4-yl)acrylaldehyd

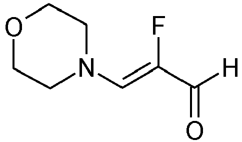

### Methode A:

Eine wässrige Lösung der Verbindung aus Beispiel 4, (max. 251,5 mmol) wurde auf 75°C erhitzt. Anschließend wurden 43,8 g (503 mmol) Morpholin und 76,3 g (755 mmol) Triethylamin zugetropft. Es wurde 2h bei 75°C gerührt, auf 23°C gekühlt und 290 ml Dichlormethan und 100 ml Triethylamin zugegeben. Die Phasen wurden getrennt, die wässrige Phase wurde mit einer Mischung aus 290 ml Dichlormethan und 100 ml Triethylamin gewaschen, die vereinigten organischen Phasen wurden filtriert, mit 250 ml ges. wässriger Kaliumcarbonatlösung gewaschen und bei 40°C am Rotationsverdampfer eingeengt. Es wurden 50 ml Toluol zugegeben und weiter aufkonzentriert. Man erhielt 34,2 g (81,9 % d. Th.) der Titelverbindung.

### Methode B:

Ein Gemisch von 43,8 g (503 mmol) Morpholin und 76,3 g (755 mmol) Triethylamin wurde auf 75°C erhitzt und eine wässrige Lösung der Verbindung aus Beispiel 4, (max. 251,5 mmol) innerhalb 25 min zugetropft. Anschließend wurde 2h bei 75°C gerührt, auf 23°C gekühlt und 290 ml Dichlormethan und 100 ml Triethylamin zugegeben. Das Gemisch wurde filtriert, die Phasen wurden getrennt, die wässrige Phase wurde mit einer Mischung aus 290 ml Dichlormethan und 100 ml Triethylamin gewaschen, die vereinigten organischen Phasen wurden mit 250 ml ges. wässriger Kaliumcarbonatlösung gewaschen und bei 40°C am Rotationsverdampfer eingeengt. Es wurden 50 ml Toluol zugegeben und weiter aufkonzentriert. Man erhielt 35,3 g (83,4 % d. Th.) der Titelverbindung.
¹H-NMR (500 MHz, CDCl₃): δ = 3.51 - 3.60 (m, 4 H) 3.72 - 3.83 (m, 4 H) 6.16 (d, *J*=27.1 Hz, 1 H) 8.59 (d, *J*=18.9 Hz, 1 H) ppm.

### Methode C:

Ein Gemisch von 30,2 g (345,3 mmol) Morpholin und 52,5 g (518,0 mmol) Triethylamin wurde auf 75°C erhitzt und bei 75° - 80°C die wässrige Lösung der Verbindung aus Beispiel 4, Methode B (max. 172,7 mmol) zugetropft. Es wurde 2h unter Rückfluß gerührt, auf 23°C gekühlt und mit 100 ml Dichlormethan gewaschen. Die wässrige Phase wurde zweimal mit einer Mischung aus 100 ml Dichlormethan und 15 ml Triethylamin gewaschen, die vereinigten organischen Phasen wurden mit 85 ml ges. wässriger Kaliumcarbonatlösung gewaschen und bei 45° - 50°C im Vakuum aufkonzentriert. Es wurden 120 ml Toluol zugegeben und 60 ml Toluol abdestilliert. Die Suspension wurde über Nacht bei Raumtemperatur gerührt, das Produkt wurde abgesaugt und im Vakuumtrockenschrank bei 50°C unter leichtem Stickstoffstrom getrocknet. Man erhielt 19,2 g (68,3 % d. Th.) der Titelverbindung.

### Gegenstand der Offenbarung ist Beispiel 6

### Ethyl-5-fluor-1-(2-fluorbenzyl)-1H-pyrazolo[3,4-b]pyridin-3-carboxylat

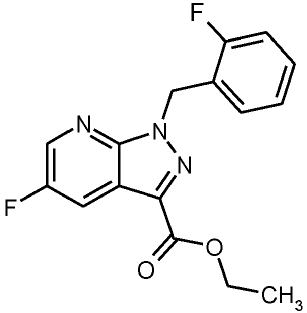

### Methode A:

22,3 g (84,8 mmol) Ethyl-5-amino-1-(2-fluorbenzyl)-1H-pyrazol-3-carboxylat (Darstellung beschrieben für Beispiel 20A in WO 00/06569) wurden in 59,5 ml Ethanol vorgelegt und bei RT 11,0 ml (169,6 mmol) Methansulfonsäure, 9,0 g (212,1 mmol) Lithiumchlorid und 15,0g (84,8 mmol) der Verbindung aus Beispiel 5 zugegeben. Das Gemisch wurde 4,5h unter Rückflusstemperatur gerührt. Nach Abkühlen auf Raumtemperatur wurde das Produkt abgesaugt, zweimal mit 4,5 ml Ethanol gewaschen und 1h mit 325 ml Wasser verrührt. Der Feststoff wurde abgesaugt, zweimal mit 11,5 ml Wasser gewaschen und im Vakuumtrockenschrank bei 50°C unter leichtem Stickstoffstrom getrocknet. Man erhielt 21,8 g (81,0 % d. Th.) der Titelverbindung.
MS (ESIpos): m/z = 318 (M+H)⁺
¹H-NMR (400 MHz, DMSO-d₆): δ = 1.37 (t, 3H), 4.40 (q, 2H), 5.86 (s, 2H), 7.15 - 7.27 (m, 3H), 7.36 - 7.41 (m, 1H), 8.25 (d, 1H), 8.78 (s br., 1H) ppm.

### Methode B:

27,0 g (635,2 mmol) Lithiumchlorid und 42,2g (254,1 mmol) der Verbindung aus Beispiel 5 wurden in 75 ml Ethanol vorgelegt und auf Rückflusstemperatur erhitzt. Bei dieser Temperatur wurde eine Lösung von 66,9 g (254,1 mmol) Ethyl-5-amino-1-(2-fluorbenzyl)-1H-pyrazol-3-carboxylat (Darstellung beschrieben für Beispiel 20A in WO 00/06569) und 33,0 ml (508,2 mmol) Methansulfonsäure in 180 ml Ethanol innerhalb 10 min zugegeben. Das Gemisch wurde 2h unter Rückflusstemperatur gerührt, anschließend wurden 120 ml Isopropanol zugegeben, auf 62°C gekühlt, mit 0,6g der Titelverbindung angeimpft und innerhalb 4h auf 5°C gekühlt. Das Produkt wurde abgesaugt, mit 120 ml Isopropanol verrührt, abgesaugt, mit 180 ml Wasser gewaschen, 0,5h mit 300 ml Wasser verrührt, abgesaugt, mit 300 ml Wasser gewaschen und im Vakuumtrockenschrank bei 50°C unter leichtem Stickstoffstrom getrocknet. Man erhielt 65,1 g (80,7 % d. Th.) der Titelverbindung.

### Methode C:

5,42 g (20,6 mmol) Ethyl-5-amino-1-(2-fluorbenzyl)-1H-pyrazol-3-carboxylat (Darstellung beschrieben für Beispiel 20A in WO 00/06569) wurden in 20 ml Ethanol vorgelegt und 1,5g (41,1 mmol) Chlorwasserstoff eingeleitet. Diese Lösung wurde innerhalb 10 min zu 3,42g (20,6 mmol) der Verbindung aus Beispiel 5 in 50 ml Ethanol bei Rückflusstemperatur zudosiert. Das Gemisch wurde 2h unter Rückflusstemperatur gerührt, anschließend wurden 10 ml Isopropanol zugegeben und auf 5°C gekühlt. Das Produkt wurde abgesaugt, mit 10 ml Isopropanol gewaschen und im Vakuumtrockenschrank bei 50°C unter leichtem Stickstoffstrom getrocknet. Man erhielt 4,84 g (74,2 % d. Th.) der Titelverbindung.

### Gegenstand der Offenbarung ist Beispiel 7

### 5 -Fluor-1-(2-fluorbenzyl)-1H-pyrazolo [3,4-b]pyridin-3 -carboxamid

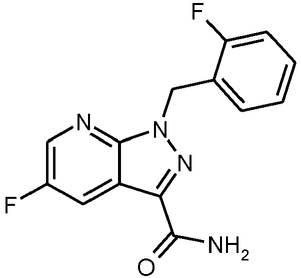

Zu 7,0 g (22,1 mmol) der unter Beispiel 6 erhaltenen Verbindung wurden 10 ml Ethanol, 14,9 ml (441,2 mmol) Formamid und 3,6 g (66,2 mmol) Natriummethylatlösung in Methanol (30%ig) gegeben. Das Reaktionsgemisch wurde auf 95° - 100°C erhitzt und die Leichtsieder dabei abdestilliert. Es wurde in 1,5 h auf 125°C gerührt, 30 ml Wasser zugegeben, auf Raumtemperatur gekühlt und 1h gerührt. Der ausgefallene Feststoff wurde abgesaugt, dreimal mit je 8,5 ml Wasser gewaschen und im Vakuumtrockenschrank bei 45°C unter leichtem Stickstoffstrom getrocknet. Man erhielt 6,2 g (97,5 % d. Th.) der Titelverbindung.
MS (ESIpos): m/z = 289 (M+H)⁺
¹H-NMR (400 MHz, DMSO-d₆): δ = 5.87 (s, 2H), 7.12 - 7.26 (m, 3H), 7.34 - 7.40 (m, 1H), 7.60 (s br., 1H), 7.87 (s br., 1H), 8.28 (dd, 1H), 8.72 (dd, 1H) ppm.

### Gegenstand der Offenbarung ist Beispiel 8

### 5 -Fluor-1-(2-fluorbenzyl)-1H-pyrazolo [3,4-b]pyridin-3-carbonitril

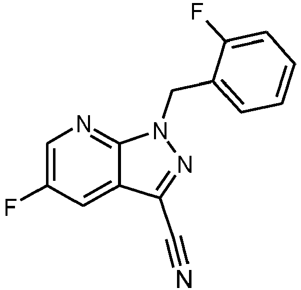

17,3 g (60,0 mmol) der unter Beispiel 7 erhaltenen Verbindung wurden in 40,5 ml Sulfolan und 5,4 ml Acetonitril auf 103° - 107°C erhitzt. Danach wurden unter Rühren 6,9 g (45,0 mmol) Phosphoroxychlorid langsam zugetropft, der Tropftrichter wurde mit 2,8 ml Acetonitril nachgespült, anschliessend wurde 1,5 h bei 107°C bis zum vollständigen Umsatz (HPLC) gerührt. Danach wurde auf Raumtemperatur abgekühlt, es wurden 2,8 ml Sulfolan/Acetonitril (5:1 vol/vol) und anschließend 17,8 ml Wasser zugetropft. Es wurde 0,5 h gerührt, eine Lösung aus 9,4 g Ammoniakwasser (28%ig) in 22,7 ml Wasser zugetropft und weitere 2h gerührt. Der ausgefallene Feststoff wurde abgesaugt, dreimal mit je 20,5 ml Wasser gewaschen und im Vakuumtrockenschrank bei 50°C unter leichtem Stickstoffstrom getrocknet. Man erhielt 14,7 g (91,9 % d. Th.) der Titelverbindung.
MS (ESIpos): m/z = 271 (M+H)⁺
¹H-NMR (400 MHz, DMSO-d₆): δ = 5.87 (s, 2H), 7.17 - 7.42 (m, 4H), 8.52 (dd, 1H), 8.87 (dd, 1H) ppm.

### Gegenstand der Offenbarung ist Beispiel 9

### 5 -Fluor-1-(2-fluorbenzyl)-1H-pyrazolo [3,4-b]pyridin-3 -carboximidamid-Hydrochlorid

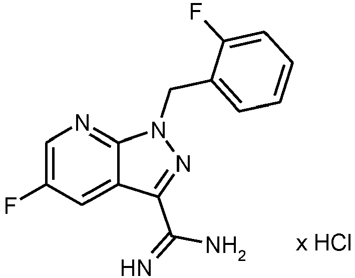

406,0 g (1,50 mol) der Verbindung aus Beispiel 8 wurden in 2,08 L Ethanol suspendiert. Anschließend wurden 54,1 g (0,30 mol) Natriummethanolat in Methanol (30%ig) zugegeben und über Nacht bei Raumtemperatur gerührt. Es wurden 88,4 g (1,65 mol) Ammoniumchlorid zugegeben, auf 65°C erhitzt und 3,5 h bei 65°C gerührt. Die Lösemittel wurden abdestilliert und der Rückstand mit 1,6 L Ethylacetat über Nacht gerührt. Der ausgefallene Feststoff wurde abgesaugt, zweimal mit je 140 ml Ethylacetat gewaschen und im Vakuumtrockenschrank bei 50°C unter leichtem Stickstoffstrom getrocknet. Man erhielt 441,4 g (90,7 % d. Th.) der Titelverbindung.
MS (ESIpos): m/z = 288 (M+H)⁺
¹H-NMR (400 MHz, DMSO-d₆): δ = 5.90 (s, 2H), 7.15 - 7.20 (m, 1H), 7.22 - 7.28 (m, 1H), 7.29 - 7.35 (m, 1H), 7.36 - 7.43 (m, 1H), 8.48 (dd, 1H), 8.86 (dd, 1H), 9.35 (br. s, 3H) ppm.

### Gegenstand der Offenbarung ist Beispiel 10

### [(E)-phenyldiazenyl]malononitril

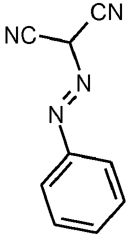

### Methode A:

Zu 1525 ml Wasser und 117,5 g (1,26 mol) Anilin wurden bei 0° - 5°C 262g konz. Salzsäure (2,59 mol) und 117,5 ml Wasser getropft. Anschliessend wurde eine Lösung von 87,1 g (1,26 mol) Natriumnitrit in 222,5 ml Wasser in 1h zugetropft, es wurde mit 60 ml Wasser nachgespült und 15 min bei 0° - 5°C gerührt. Danach wurde bei dieser Temperatur innerhalb 45 min eine Lösung aus 131,4 g (1,60 mol) Natriumacetat in 665 ml Wasser (19 ml) zugetropft, es wurde mit 60 ml Wasser nachgespült und innerhalb 1 h eine Lösung aus 83,4 g (1,26 mol) Malonsäuredinitril in 233 ml Ethanol zugetropft. Es wurde mit 68,5 ml Ethanol nachgespült und 2 h bei 0° - 5°C gerührt. Der gelbe Feststoff wurde abgesaugt und dreimal mit je 625 ml Wasser und mit 488 ml kaltem Toluol gewaschen. Der noch feuchte Rückstand wurde in 872 g DMF gelöst. Man erhielt 1117,0 g DMF-Lösung der Titelverbindung.

### Methode B:

Zu 508,5 ml Wasser und 39,2 g (0,42 mol) Anilin wurden bei 0° - 5°C 87,4g konz. Salzsäure (0,86 mol) und 39,5 ml Wasser getropft. Anschliessend wurde eine Lösung von 29,0 g (0,42 mol) Natriumnitrit in 74,5 ml Wasser in 1h zugetropft, es wurde mit 20 ml Wasser nachgespült und 15 min bei 0° - 5°C gerührt. Danach wurde bei dieser Temperatur innerhalb 45 min eine Lösung aus 43,8 g (0,54 mol) Natriumacetat in 221,5 ml Wasser zugetropft, es wurde mit 20 ml Wasser nachgespült und innerhalb 1 h eine Lösung aus 27,8 g (0,42 mol) Malonsäuredinitril in 77,5 ml Ethanol zugetropft. Es wurde mit 23 ml Ethanol nachgespült und 2 h bei 0° - 5°C gerührt. Der gelbe Feststoff wurde abgesaugt und dreimal mit je 208,5 ml Wasser und mit 162,5 ml kaltem Toluol gewaschen. Es wurden 103,1g Feuchtprodukt erhalten. 13,8g des Feuchtprodukts wurden in 13,9 g Sulfolan gelöst. Man erhielt 27,7 g Sulfolan-Lösung der Titelverbindung.

### Gegenstand der Offenbarung ist Beispiel 11

### 2-[5-Fluor-1-(2-fluorbenzyl)-1H-pyrazolo[3,4-b]pyridin-3-yl]-5-[(E)-phenyldiazenyl]pyrimidin-4,6-diamin

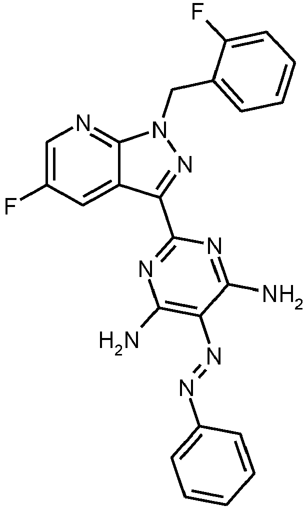

### Methode A:

In 1059 ml DMF wurden 448,2 g ( 1,38 mol) der Verbindung aus Beispiel 9 suspendiert. Es wurde auf 85°C erhitzt und bei dieser Temperatur 212 ml (1,52 mol) Triethylamin zugetropft. Anschließend wurden innerhalb 20 min 1751 g der DMF-Lösung aus Beispiel 10 zugetropft, es wurde mit 490 ml DMF nachgespült und über Nacht bei 100°C nachgerührt. Das Reaktionsgemisch wurde auf RT abgekühlt, es wurden 656 ml Wasser zugetropft und 0,5 h bei RT gerührt, dann auf 0° - 5°C gekühlt und 1h nachgerührt. Der Feststoff wurde abgesaugt, zweimal mit je einer Lösung aus 1443 g Wasser und 236 g Methanol und anschließend mit 586 ml Methanol gewaschen, trockengesaugt und im Vakuumtrockenschrank bei 50°C unter leichtem Stickstoffstrom getrocknet. Man erhielt 522,2 g (82,5 % d. Th.) der Titelverbindung.
¹H-NMR (400 MHz, DMSO-d₆): δ = 5.84 (s, 2 H) 7.14 - 7.28 (m, 3 H) 7.34 - 7.41 (m, 2 H) 7.46 - 7.52 (m, 2 H) 7.95 (br. s, 2 H) 8.02 (dd, 2 H) 8.50 (br. s, 2 H) 8.70 - 8.73 (m, 1 H) 9.02 - 9.06 (m, 1 H) ppm.

### Methode B:

In 72 ml DMF wurden 30,0 g (92,7 mmol) der Verbindung aus Beispiel 9 suspendiert. Es wurde auf 100°C erhitzt und bei dieser Temperatur innerhalb 30 min ein Gemisch aus 14,2 ml (101,9 mmol) Triethylamin und 150 g der DMF-Lösung aus Beispiel 10 zugetropft. Es wurde mit 30 ml DMF nachgespült und über 20 h bei 100°C nachgerührt. Das Reaktionsgemisch wurde auf 95° - 90°C abgekühlt, innerhalb 10 min wurden 24 ml Wasser zugetropft, dann innerhalb 1,5 h auf 0° - 5°C gekühlt und 1h nachgerührt. Der Feststoff wurde abgesaugt, mit einer Lösung aus 60 g Wasser und 60g Dimethylformamid gewaschen, zweimal mit je einer Lösung aus 50g Wasser und 50g Methanol und anschließend mit 40 ml Methanol gewaschen, trockengesaugt und im Vakuumtrockenschrank bei 50°C unter leichtem Stickstoffstrom getrocknet. Man erhielt 35,5 g (83,7 % d. Th.) der Titelverbindung.

### Methode C:

In 15,6 ml Sulfolan wurden 11,7 g (36,0 mmol) der Verbindung aus Beispiel 9 suspendiert. Es wurde auf 100°C erhitzt und bei dieser Temperatur innerhalb 35 min ein Gemisch aus 5,5 ml (39,6 mmol) Triethylamin und 27,7 g der Sulfolan-Lösung aus Beispiel 10 Methode B zugetropft. Es wurde mit 2 ml Sulfolan nachgespült und über 2,5 h bei 100°C nachgerührt. Das Reaktionsgemisch wurde auf 60°C abgekühlt, 90 ml Isopropanol zugetropft, dann innerhalb 15 min auf 0° - 5°C gekühlt und 2,5h nachgerührt. Der Feststoff wurde abgesaugt, dreimal mit je 50 g Wasser und 24 ml Isopropanol gewaschen, trockengesaugt und im Vakuumtrockenschrank bei 50°C unter leichtem Stickstoffstrom getrocknet. Man erhielt 14,2 g (85,9 % d. Th.) der Titelverbindung.

### Gegenstand der Offenbarung ist Beispiel 12

### 2-[5-Fluor-1-(2-fluorbenzyl)-1H-pyrazolo[3,4-b]pyridin-3-yl]pyrimidin-4,5,6-triamin

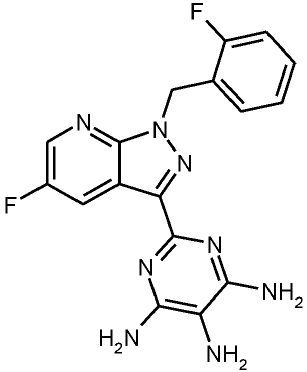

### Methode A:

In 1,82 L DMF wurden 182,0 g (0,39 mol) der Verbindung aus Beispiel 11 vorgelegt und anschliessend 4,2 g Palladium (5%ig auf Kohle, 50% wasserfeucht) zugefügt. Es wurde unter Rühren über Nacht bei 60°C und 60 bar Wasserstoffdruck hydriert. Der Ansatz wurde über Kieselgur filtriert und mit 150 ml DMF und danach mit 150 ml Methanol nachgewaschen und bis zu einem Gewicht von 425 g Destillationsrückstand bei 60° - 70°C eingeengt. Der Rückstand wurde auf 75° - 80°C erwärmt, bei dieser Temperatur wurden 300 ml Methanol zugetropft und 15 min gerührt. Innerhalb 1 h wurde auf RT gekühlt, anschließend 1290 ml Wasser zugetropft und über Nacht gerührt. Der Feststoff wurde abgesaugt, zweimal mit je 500 ml Wasser gewaschen, trockengesaugt und im Vakuumtrockenschrank bei 50°C unter leichtem Stickstoffstrom getrocknet. Man erhielt 159,7 g der Titelverbindung. Das Produkt hat einen Gehalt von 73,7 Gew.-% und 12,4 Gew.-% DMF (80,3 % d. Th.) und wurde so in die Folgestufe eingesetzt. Je nach Intensität der Wasserwäsche lag der Gehalt an DMF im Bereich von 10 - 17 Gew.-%.

### Methode B:

25,0g des DMF-haltigen Feststoffs aus Methode A wurden in 220 ml Wasser suspendiert und über eine Nutsche abgesaugt. Es wurde viermal mit je 100 ml 95°C heissem Wasser auf der Nutsche gewaschen, trockengesaugt und im Vakuumtrockenschrank bei 50°C unter leichtem Stickstoffstrom getrocknet. Man erhielt 21,2 g der DMF-freien Titelverbindung.
MS (ESIpos): m/z = 369 (M+H)⁺

Für analytische Zwecke wurde eine Probe mittels Kieselgelfiltration aufgereinigt:
¹H-NMR (400 MHz, DMSO-d₆): δ = 4.04 (br. s, 2 H) 5.75 (s, 2 H) 5.86 (br. s, 4 H) 7.10 - 7.26 (m, 3 H) 7.32 - 7.39 (m, 1 H) 8.61 - 8.64 (m, 1 H) 8.85 (dd, 1 H) ppm.

### Beispiel 13

### Methyl-{4,6-diamino-2-[5-fluor-1-(2-fluorbenzyl)-1H-pyrazolo[3,4-b]pyridin-3-yl]pyrimidin-5-yl}carbamat

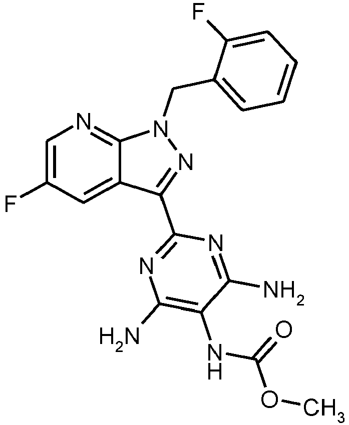

### Methode A:

In 37,9 ml Isopropanol wurden 4,0 g (77,0 Gew.-%, 8,36 mmol) der Verbindung aus Beispiel 12 auf 35°C erwärmt und anschließend 0,84 ml (10,87 mmol) Chlorameisensäuremethylester zugetropft. Es wurde 20h bei 35° - 40°C gerührt, auf 50°C erwärmt und 9,5 ml Methanol zugegeben. Anschließend wurden innerhalb 0,5 h 1,9 ml Triethylamin zugetropft, mit 1,3 ml Methanol nachgespült und 1h bei 50°C gerührt. Danach wurde die Reaktionsmischung auf RT gekühlt, 1 h bei RT gerührt, der Feststoff wurde abgesaugt, dreimal mit je 8 ml Ethanol gewaschen, trockengesaugt und im Vakuumtrockenschrank bei 50°C unter leichtem Stickstoffstrom getrocknet. Man erhielt 3,4 g Rohprodukt. 3,0 g des Rohprodukts wurden in 8 ml DMSO 5 min verrührt, es wurden 13,0 ml Ethylacetat und 50 mg Aktivkohle zugegeben und 15 min unter Rückfluß (84°C) erhitzt. Die Suspension wurde heiß filtriert und der Filterrückstand mit 1,9 ml Ethylacetat¹⁾ gewaschen. 60 ml Ethylacetat und 16 ml Ethanol wurden auf 60°C erwärmt, die vereinigten Filtrate wurden zugetropft und 1,5 h bei 60°C nachgerührt. Die Suspension wurde innerhalb 25 min auf RT gekühlt, 1,5 h nachgerührt weiter auf 0° - 5°C gekühlt, und 1 h nachgerührt. Der Feststoff wurde abgesaugt, zweimal mit je 6,4 ml Ethylacetat gewaschen, trockengesaugt und im Vakuumtrockenschrank bei 50°C unter leichtem Stickstoffstrom getrocknet. Man erhielt 2,2 g (70,0 % d. Th.) der Titelverbindung.
MS (ESIpos): m/z = 427 (M+H)⁺
¹H-NMR (400 MHz, DMSO-d₆): δ = 3.62 (br s, 3H), 5.79 (s, 2H), 6.22 (br s, 4H), 7.10 - 7.19 (m, 2H), 7.19 - 7.26 (m, 1H), 7.32 - 7.40 (m, 1H), 7.67 und 7.99 (2 br s, 1H), 8.66 (m, 1H), 8.89 (dd, 1H) ppm.
**1) Nach dem beschriebenen Herstellverfahren wird an dieser Stelle das Di-Dimethylsulfoxid-Solvat erhalten,** welches in Tabellen 2 und 4 anhand der Reflexe des Röntgendiffraktogramms und Banden des IR-Spektrum charakterisiert ist.

Das Di-Dimethylsulfoxid-Solvat der Verbindung der Formel (I) hat den Vorteil einer deutlich besseren Filtrierbarkeit als die Substanz im Stand der Technik. Weiterhin führt das Herstellverfahren über das Di-Dimethylsulfoxid-Solvat der Verbindung der Formel (I) zu einer sehr hohen Reinheit der Verbindung der Formel (I).

### Methode B:

In 37,9 ml Isopropanol wurden 4,0 g (10,8 mmol) der Verbindung aus Beispiel 12 Methode B auf 35°C erwärmt und anschließend 1,1 mL (14,1 mmol) Chlorameisensäuremethylester zugetropft. Es wurde 16,5h bei 35° - 40°C gerührt, auf RT gekühlt und 2,1 ml Ammoniakwasser (28%) zugegeben. Anschließend wurden 4,2 ml Wasser zugegeben und 2,5 h gerührt. Der Feststoff wurde abgesaugt, zweimal mit je 5 ml Wasser gewaschen, trockengesaugt und im Vakuumtrockenschrank bei 50°C unter leichtem Stickstoffstrom getrocknet. Man erhielt 4,4 g Rohprodukt.

### Methode C:

In 37,9 ml Isopropanol wurden 4,0 g (10,8 mmol) der Verbindung aus Beispiel 12 Methode B auf 35°C erwärmt und anschließend 1,1 mL (14,1 mmol) Chlorameisensäuremethylester zugetropft. Es wurde 16,5h bei 35° - 40°C gerührt, und bei 50°C 9,5 ml Methanol zugegeben. Anschließend wurden 2,42 ml Triethylamin in 20 min zugetropft mit 1,3 ml Methanol nachgespült und 1h bei 50°C gerührt. Danach wurde die Reaktionsmischung auf RT gekühlt, 1 h bei RT gerührt, der Feststoff wurde abgesaugt, dreimal mit je 8 ml Methanol gewaschen, trockengesaugt und im Vakuumtrockenschrank bei 50°C unter leichtem Stickstoffstrom getrocknet. Man erhielt 4,3 g Rohprodukt.

### Methode D:

6,9 g des Rohprodukts wurden in 18,4 ml DMSO 5 min verrührt, es wurden 30,0 ml Ethylacetat und 115 mg Aktivkohle zugegeben und 15 min unter Rückfluß (84°C) erhitzt. Die Suspension wurde heiß filtriert und der Filterrückstand mit 4,4 ml Ethylacetat gewaschen. 138 ml Ethylacetat wurden auf 50°C erwärmt, die vereinigten Filtrate wurden zugetropft und 1 h bei 45° - 50°C nachgerührt. Die Suspension wurde innerhalb 1,5 h auf 0° - 5°C gekühlt und 1 h nachgerührt. Der Feststoff wurde abgesaugt, zweimal mit je 14,8 ml Ethylacetat gewaschen und 1 h trockengesaugt. Es wurden 6,4g de Di-Dimethylsulfoxid-Solvats als Feuchtprodukt¹⁾ erhalten.

### Methode E:

2,0 g des Di-Dimethylsulfoxid-Solvats wurden in 40 ml Ethylacetat und 11,1 ml Ethanol 17 h bei Rückflusstemperatur gerührt, auf RT abgekühlt und 1h nachgerührt. Der Feststoff wurde abgesaugt, viermal mit je 1,4 ml Ethylacetat gewaschen und im Vakuumtrockenschrank bei 50°C unter leichtem Stickstoffstrom getrocknet. Man erhielt 1,4 g der Titelverbindung vorliegend in der Modifikation I.

### Methode F:

0,5 g des Di-Dimethylsulfoxid-Solvats wurden in 12,5 ml Lösungsmittel 17 h bei Rückflusstemperatur gerührt, auf RT abgekühlt und 1h nachgerührt. Der Feststoff wurde abgesaugt, mit 2 ml Lösungsmittel gewaschen und 30 min trockengesaugt. Man erhielt 0,3 g der Titelverbindung vorliegend in der Modifikation I.

Es wurden folgende Lösungsmittel verwendet:
1.) 9 ml Ethylacetat/3,5 ml Ethanol/0,3 ml Wasser
2.) 12,5 ml Isopropanol
3.) 12,5 ml Isopropanol/0,3 mlWasser
4.) 12,5 ml Methanol
5.) 12,5 ml Methanol/0,3 ml Wasser
6.) 12,5 ml Acetonitril
7.) 12,5 ml Aceton
8.) 12,5 ml Tetrahydrofuran,
9.) 12,5 ml Methyl-tert.-butylether

In Tabelle 1 sind die Reflexe der Röntgendiffraktogramms aufgezeigt. Tabelle 3 zeigt die Banden des IR-Spektrums.

Die Verbindung (I) in der kristallinen Modifikation I zeichnet sich durch eine höhere Stabilität und insbesondere dadurch aus, dass sie im Mikronisierprozess stabil ist und somit keine Umwandlung und Rekristallisation stattfindet.

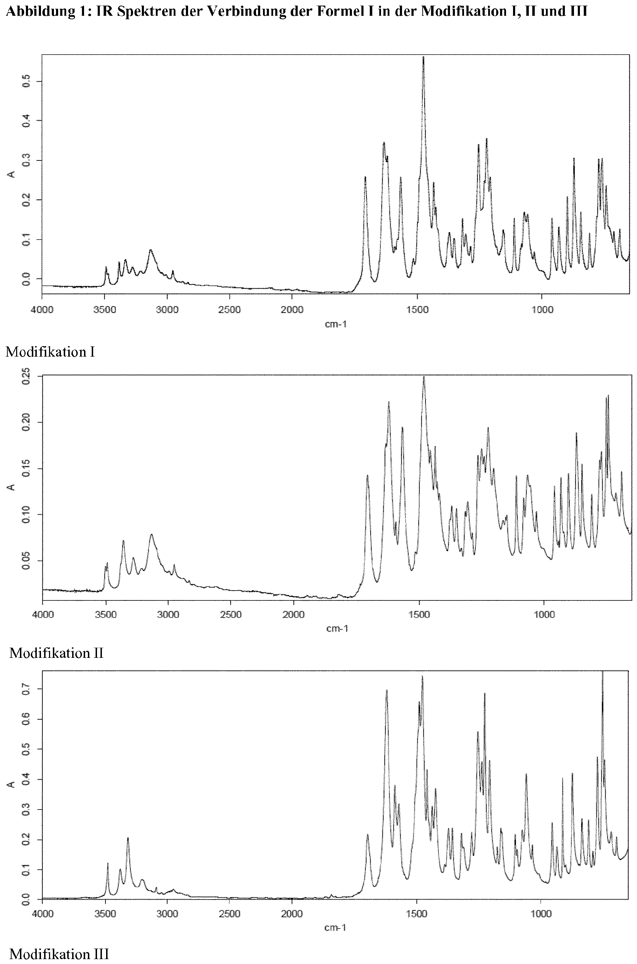

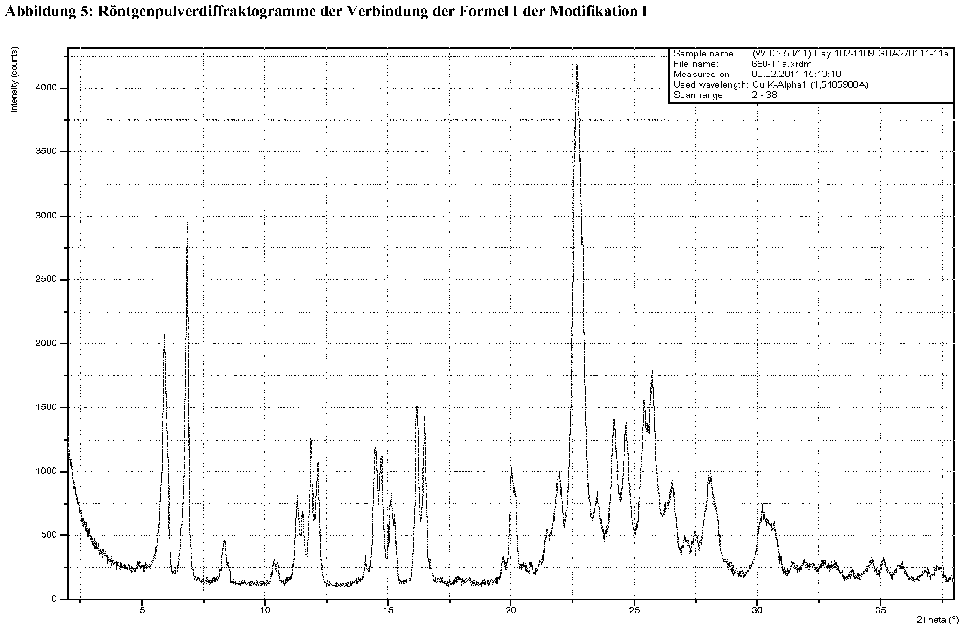

Die Verbindung der Formel (I) lässt sich nach oben beschrieben Verfahren herstellen. Dabei wird die Verbindung der Formel (I) in einer Kristallmodifikation erhalten, die im Folgenden als Modifikation I bezeichnet wird. Modifikation I hat einen Schmelzpunkt von 257°C und ein charakteristisches Röntgendiffraktogramm, gekennzeichnet durch die Reflexe (2 Theta) 5.9, 6.9, 16.2, 16.5, 24.1 und 24.7 sowie ein charakteristisches IR-Spektrum, gekennzeichnet durch die Bandenmaxima (in cm⁻¹) 1707, 1633, 1566, 1475, 1255 und 1223 (Tabelle 1 und 3, Abbildung 1 und 5).

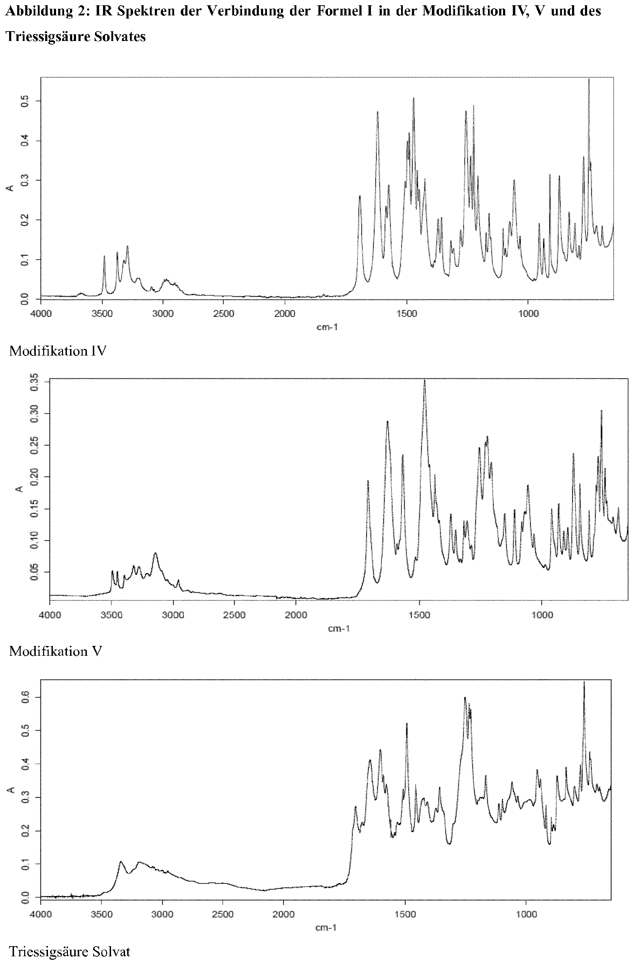

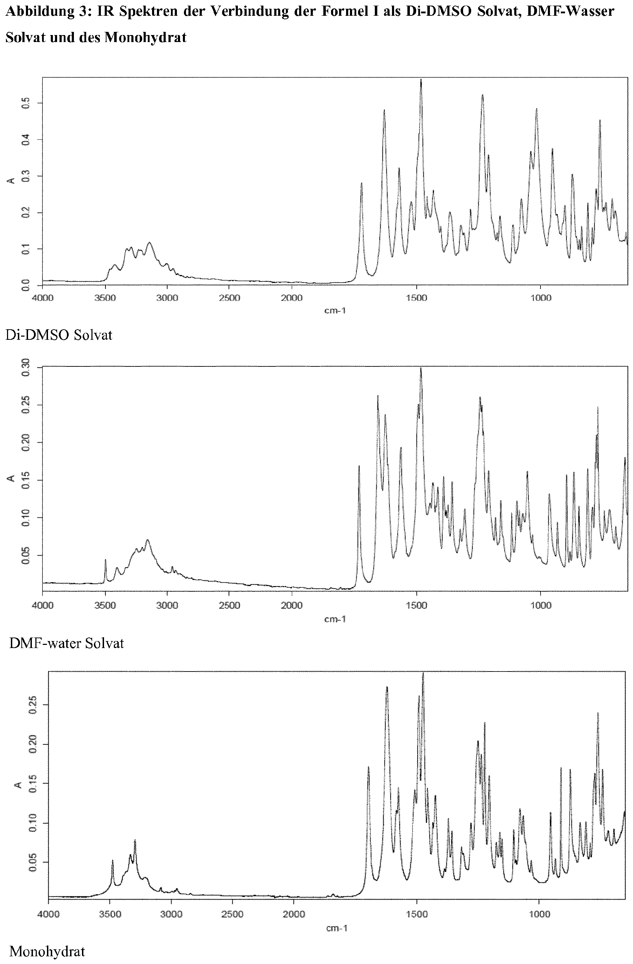

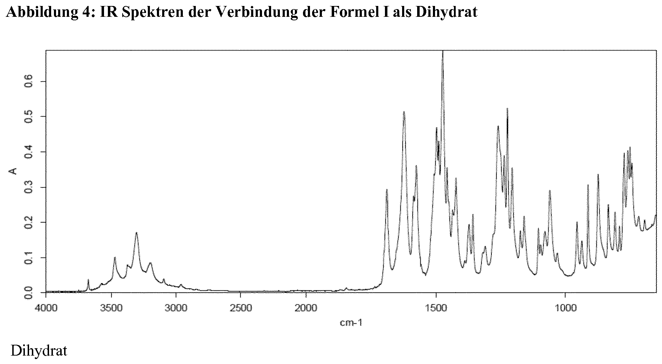

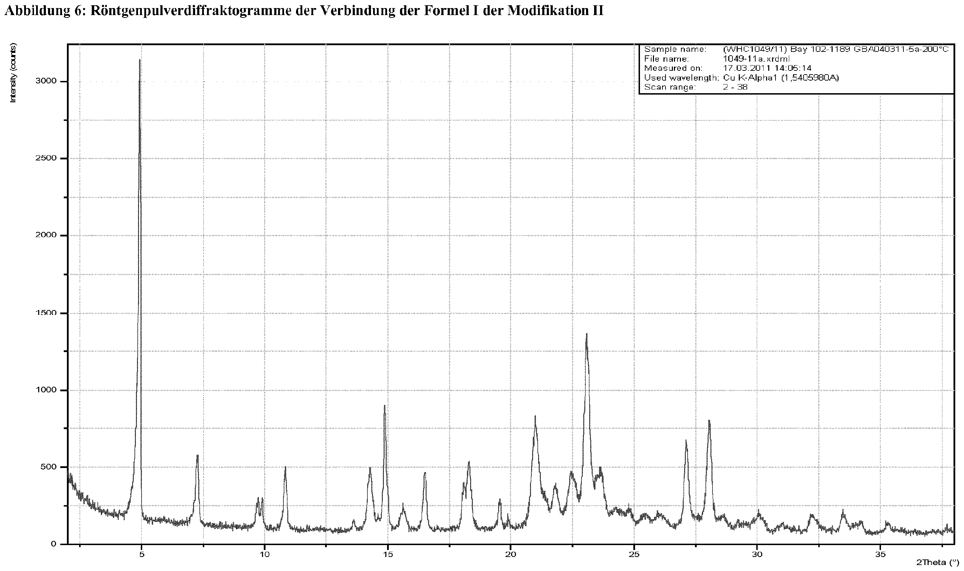

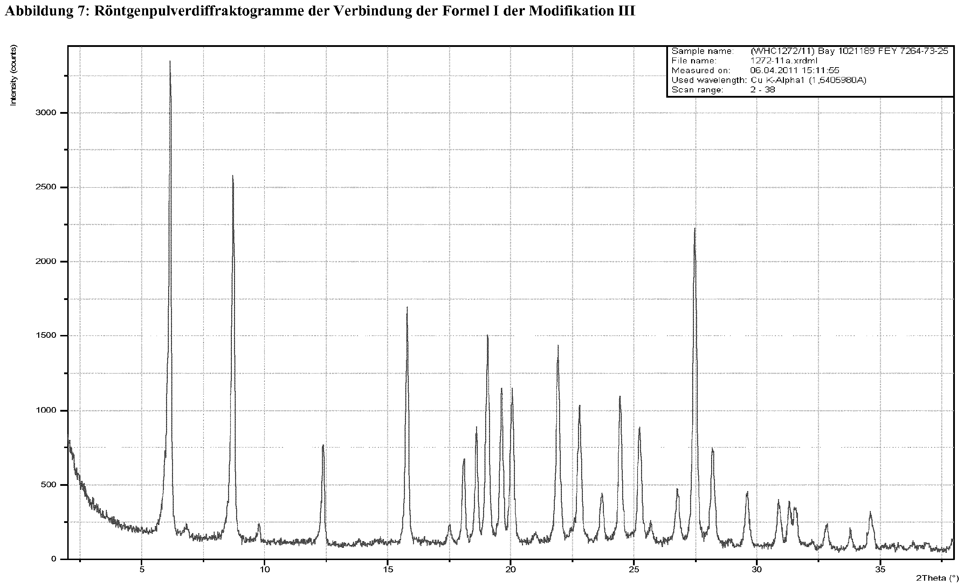

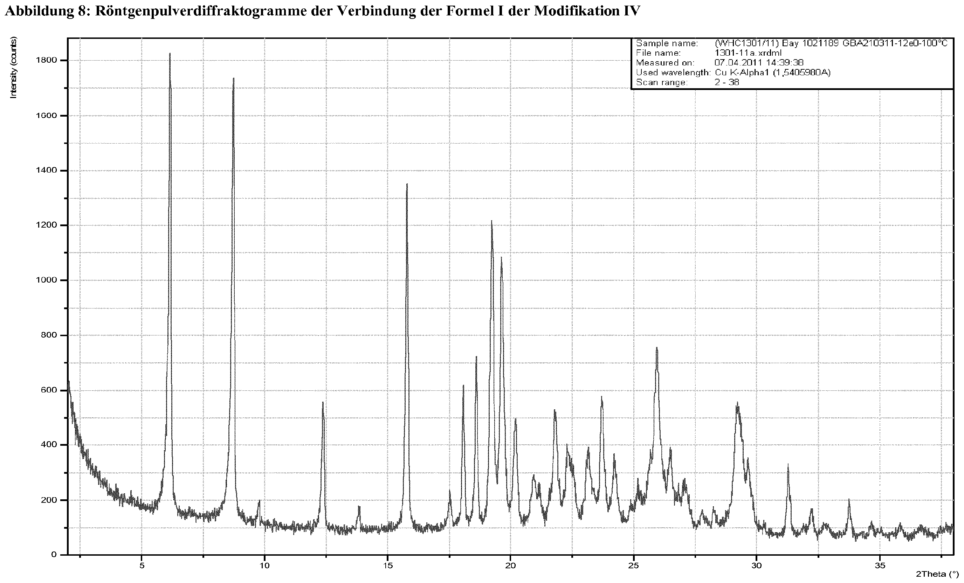

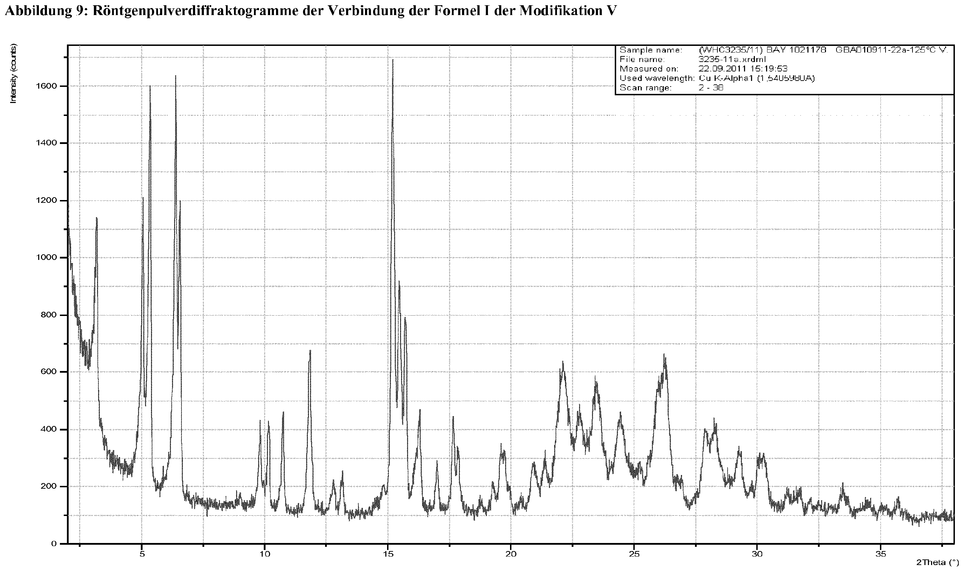

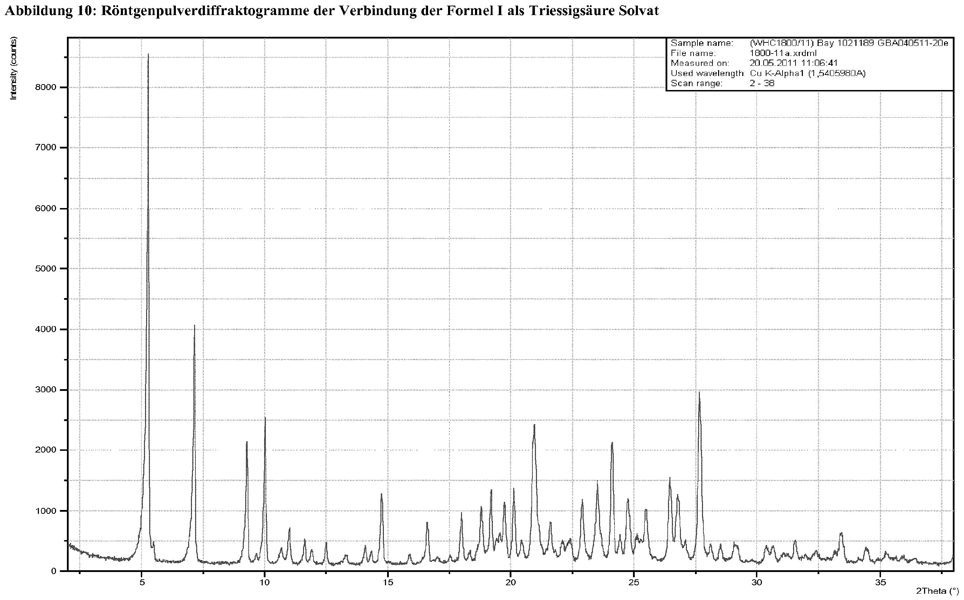

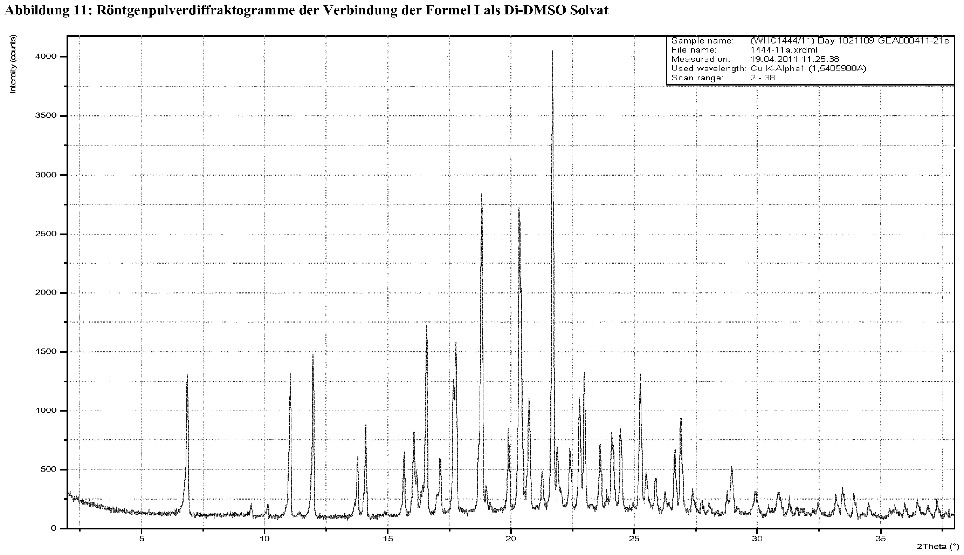

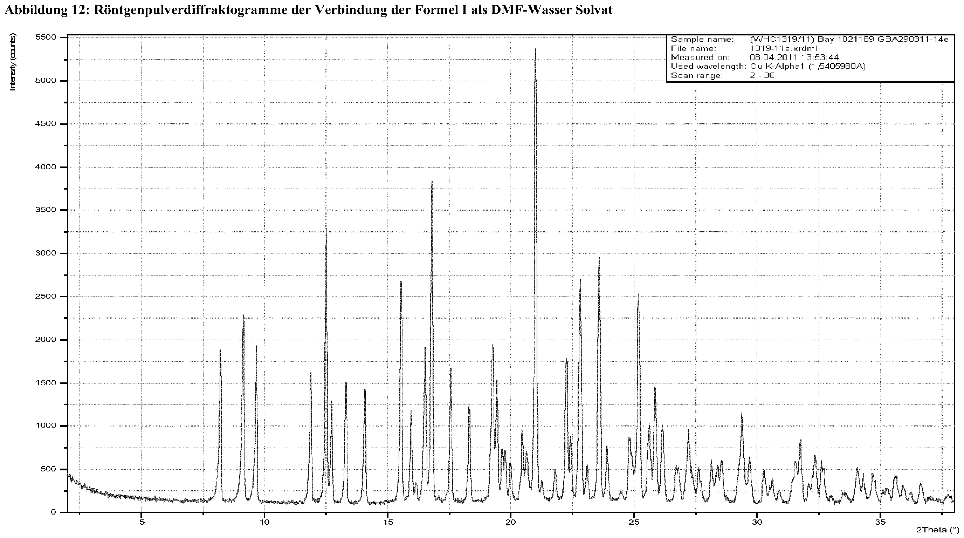

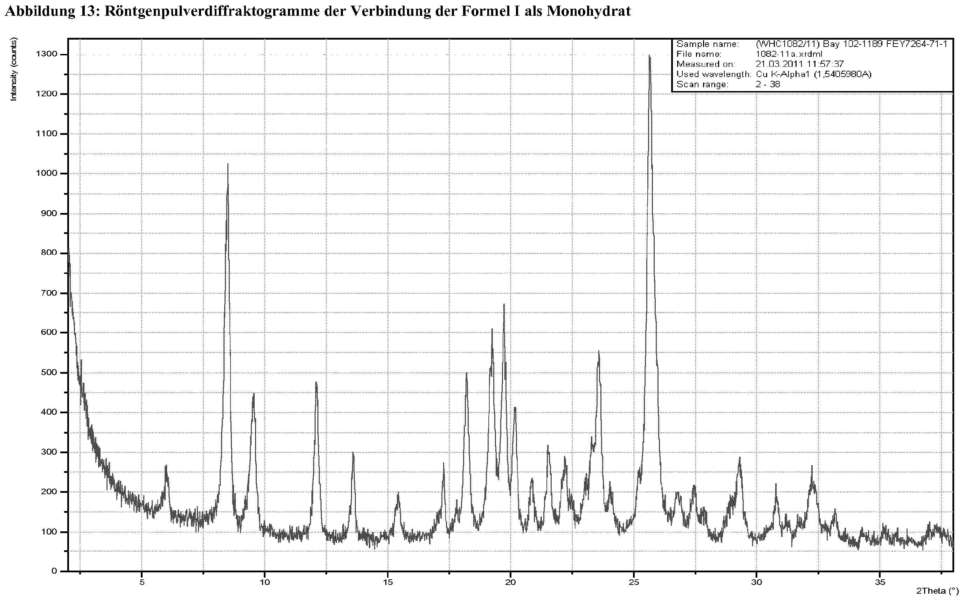

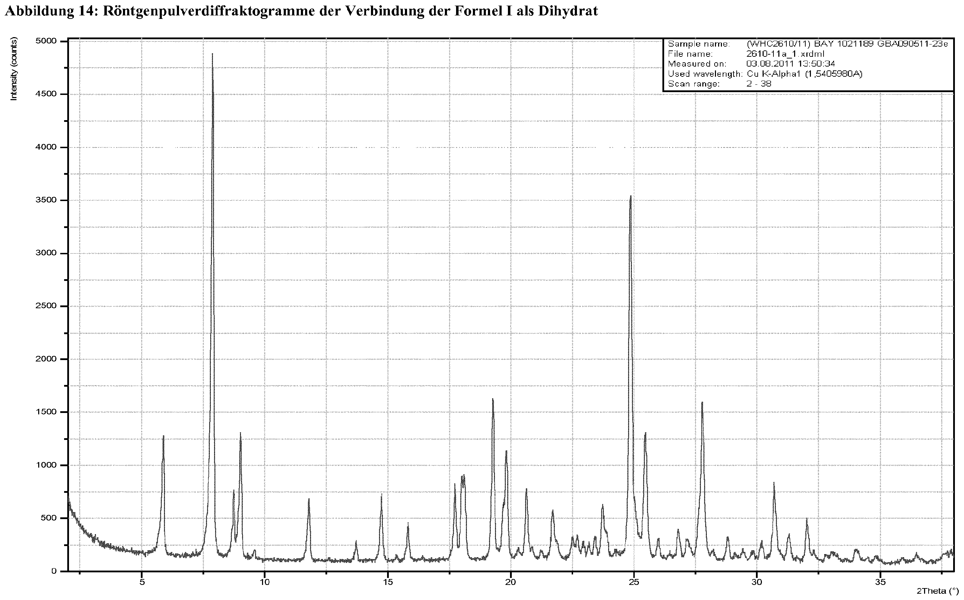

Überraschenderweise wurden vier weitere Modifikationen, ein Monohydrat, ein Dihydrat ein DMF-Wasser-Solvat und ein Di-Dimethylsulfoxid-Solvat sowie ein Tri-Essigsäure-Solvat der Verbindung der Formel (I) gefunden. Die Verbindung der Formel (I) in der Modifikation II schmilzt bei ca. 253°C, die Verbindung der Formel (I) in der Modifikation III hat einen Schmelzpunkt von ca. 127°C. Modifikation IV der Verbindung der Formel I schmilzt bei einer Temperatur von 246°C, während Modifikation V einen Schmelzpunkt von 234°C hat. Das Monohydrat enthält ca. 4.1 % Wasser, das Dihydrat enthält 7.8 % Wasser, das DMF-Wasser-Solvat enthält 13.6 % Dimethylformamid und 0.9 % Wasser, das Di-DMSO-Solvat enthält 26.8 % Dimethylsulfoxid und das Tri-Essigsäure-Solvat enthält 29.7 % Acetat. Alle genannten kristallinen Formen zeigen jeweils ein charakteristisches Röntgendiffraktogramm und IR-Spektrum (Tabelle 2 und 3, Abbildung 1 - 4, 6 - 14).

**Tabelle 1: Röntgendiffraktometrie Modifikation I bis V**

| **Reflexe** | | | | |
|---|---|---|---|---|
| Modifikation I [2 Theta] | Modifikation II [2 Theta] | Modifikation III [2 Theta] | Modifikation IV [2 Theta] | Modifikation V [2 Theta] |
| 5.9 | 4.9 | 6.2 | 6.2 | 3.2 |
| 6.9 | 7.3 | 6.8 | 8.7 | 5.1 |
| 8.3 | 9.7 | 8.7 | 12.4 | 5.4 |
| 10.4 | 9.9 | 9.8 | 15.8 | 6.4 |
| 10.5 | 10.8 | 12.4 | 18.1 | 6.6 |
| 11.3 | 14.3 | 15.8 | 18.6 | 10.2 |
| 11.6 | 14.9 | 17.5 | 19.2 | 10.7 |
| 11.9 | 15.6 | 18.1 | 19.6 | 11.8 |
| 12.2 | 16.5 | 18.6 | 20.2 | 12.8 |
| 14.5 | 18.1 | 19.1 | 20.9 | 13.2 |
| 14.7 | 18.3 | 19.6 | 21.8 | 15.2 |
| 15.1 | 19.6 | 20.1 | 22.3 | 15.5 |
| 16.2 | 21.0 | 21.0 | 23.1 | 15.7 |
| 16.5 | 21.8 | 21.9 | 23.7 | 16.3 |
| 20.0 | 22.4 | 22.8 | 24.2 | 17.0 |
| 21.9 | 23.1 | 23.7 | 26.0 | 17.7 |
| 22.7 | 23.7 | 24.5 | 26.5 | 17.9 |
| 23.5 | 27.1 | 25.3 | 29.2 | 19.6 |
| 24.1 | 28.1 | 25.7 | 31.3 | 22.1 |
| 24.7 | | 26.8 | 33.8 | 22.8 |
| 25.4 | | 27.5 | | 23.5 |
| 25.7 | | 28.2 | | 24.4 |
| 26.6 | | 29.6 | | 26.3 |
| 28.0 | | 30.9 | | 27.9 |
| 30.2 | | 31.3 | | 28.3 |
| | | 31.6 | | 29.3 |
| | | 32.8 | | 30.3 |
| | | 33.8 | | |
| | | 34.6 | | |

**Tabelle 2: Röntgendiffraktometrie Modifikation Hydrate und Solvate**

| **Reflexe** | | | | |
|---|---|---|---|---|
| Monohydrat [2 Theta] | Dihydrat [2 Theta] | DMF-Wasser-Solvat [2 Theta] | Di-DMSO-Solvat [2 Theta] | Essigsäure-Solvat [2 Theta] |
| 6.0 | 5.9 | 8.2 | 6.9 | 5.3 |
| 8.5 | 7.9 | 9.2 | 11.0 | 7.2 |
| 9.6 | 8.7 | 9.7 | 12.0 | 9.3 |
| 12.1 | 9.0 | 11.9 | 13.8 | 10.0 |
| 13.6 | 11.8 | 12.5 | 14.1 | 10.7 |
| 15.5 | 13.7 | 12.7 | 15.7 | 11.0 |
| 17.3 | 14.7 | 13.3 | 16.1 | 11.6 |
| 18.2 | 15.8 | 14.1 | 16.2 | 11.9 |
| 19.3 | 16.4 | 15.6 | 16.6 | 12.5 |
| 19.7 | 18.1 | 16.0 | 17.1 | 14.1 |
| 20.2 | 19.3 | 16.5 | 17.7 | 14.4 |
| 20.9 | 19.8 | 16.8 | 17.8 | 14.8 |
| 21.5 | 20.6 | 17.6 | 18.8 | 16.6 |
| 22.2 | 21.7 | 18.3 | 19.9 | 18.0 |
| 23.5 | 21.7 | 19.3 | 20.3 | 18.8 |
| 24.1 | 22.5 | 19.4 | 20.7 | 19.2 |
| 25.7 | 22.7 | 19.6 | 21.3 | 19.4 |
| 26.8 | 22.9 | 19.8 | 21.7 | 19.6 |
| 27.5 | 23.4 | 20.0 | 21.9 | 19.7 |
| 29.4 | 23.7 | 20.5 | 22.4 | 20.1 |
| 30.8 | 24.9 | 20.6 | 22.8 | 20.4 |
| 32.2 | 25.5 | 20.7 | 23.6 | 21.0 |
| | 26.0 | 21.0 | 24.1 | 21.6 |
| | 26.8 | 21.8 | 24.4 | 22.9 |
| | 27.1 | 22.2 | 25.2 | 23.5 |
| | 27.8 | 22.4 | **25.5** | 24.1 |
| | 28.9 | 22.8 | 25.9 | 24.4 |
| | 30.7 | 23.1 | 26.6 | 24.8 |
| | 31.3 | 23.6 | 26.9 | 25.5 |
| | 32.0 | 23.9 | 28.9 | 26.5 |
| | | 24.8 | 29.9 | 26.8 |
| | | 25.2 | 30.9 | 27.7 |
| | | 25.6 | 33.2 | 31.5 |
| | | 25.8 | 33.4 | |
| | | 26.1 | 33.9 | |
| | | 26.7 | | |
| | | 26.8 | | |
| | | 27.2 | | |
| | | 27.6 | | |
| | | 28.1 | | |
| | | 28.4 | | |
| | | 28.6 | | |
| | | 29.4 | | |
| | | 29.7 | | |
| | | 30.3 | | |
| | | 30.6 | | |
| | | 31.4 | | |
| | | 31.5 | | |
| | | 31.7 | | |
| | | 32.1 | | |
| | | 32.4 | | |
| | | 32.6 | | |
| | | 32.7 | | |
| | | 34.1 | | |
| | | 34.3 | | |
| | | 34.7 | | |
| | | 35.6 | | |
| | | 35.9 | | |
| | | 36.6 | | |

**Tabelle 3: IR-Spektren der Modifikation I bis V**

| **Bandenmaxima** | | | | |
|---|---|---|---|---|
| Modifikation I [cm⁻¹] | Modifikation II [cm⁻¹] | Modifikation III [cm⁻¹] | Modifikation IV [cm⁻¹] | Modifikation V [cm⁻¹] |
| 690 | 691 | 697 | 698 | 691 |
| 744 | 752 | 744 | 752 | 745 |
| 761 | 771 | 753 | 773 | 759 |
| 774 | 779 | 773 | 809 | 773 |
| 810 | 810 | 808 | 833 | 809 |
| 845 | 848 | 835 | 873 | 847 |
| 872 | 871 | 873 | 911 | 873 |
| 899 | 903 | 913 | 936 | 896 |
| 960 | 933 | 935 | 955 | 912 |
| 1059 | 958 | 954 | 1058 | 933 |
| 1072 | 1031 | 1034 | 1077 | 961 |
| 1112 | 1067 | 1059 | 1104 | 1033 |
| 1157 | 1082 | 1075 | 1161 | 1057 |
| 1208 | 1111 | 1103 | 1207 | 1083 |
| 1223 | 1202 | 1161 | 1225 | 1112 |
| 1255 | 1223 | 1206 | 1237 | 1152 |
| 1305 | 1249 | | 1256 | 1207 |
| 1319 | 1264 | 1237 | 1277 | 1224 |
| 1353 | 1305 | 1253 | 1317 | 1255 |
| 1370 | 1349 | 1278 | 1356 | 1305 |
| 1435 | 1368 | 1319 | 1370 | 1318 |
| 1475 | 1436 | 1355 | 1425 | 1351 |
| 1566 | 1456 | 1370 | 1457 | 1371 |
| 1620 | 1480 | 1424 | 1472 | 1436 |
| 1633 | 1566 | 1437 | 1490 | 1478 |
| 1707 | 1620 | 1458 | 1496 | 1567 |
| 2956 | 1704 | 1476 | 1573 | 1628 |
| 3130 | 2953 | 1489 | 1585 | 1707 |
| 3277 | 3132 | 1570 | 1618 | 2956 |
| 3332 | 3278 | 1587 | 1691 | 3143 |
| 3385 | 3361 | 1619 | 3208 | 3277 |
| 3490 | 3488 | 1695 | 3290 | 3319 |
| | 3503 | 3203 | 3376 | 3452 |
| | | 3315 | 3482 | 3492 |
| | | 3379 | | |
| | | 3479 | | |

**Tabelle 4: IR-Spektren der Hydrate und Solvate**

| **Bandenmaxima** | | | | |
|---|---|---|---|---|
| Monohydrat [cm⁻¹] | Dihydrat [cm⁻¹] | DMF-Wasser-Solvat [cm⁻¹] | Di-DMSO-Solvat [cm⁻¹] | Essigsäure-Solvat [cm⁻¹] |
| 696 | 745 | 662 | 713 | 709 |
| 743 | 752 | 724 | 762 | 739 |
| 761 | 760 | 745 | 778 | 762 |
| 774 | 774 | 771 | 811 | 777 |
| 810 | 809 | 812 | 873 | 801 |
| 834 | 835 | 846 | 902 | 835 |
| 873 | 874 | 867 | 953 | 872 |
| 912 | 913 | 896 | 1017 | 918 |
| 953 | 937 | 932 | 1041 | 941 |
| 1066 | 955 | 965 | 1078 | 955 |
| 1079 | 1032 | 1054 | 1111 | 1059 |
| 1104 | 1061 | 1072 | 1164 | 1099 |
| 1160 | 1080 | 1096 | 1210 | 1113 |
| 1176 | 1105 | 1117 | 1234 | 1167 |
| 1205 | 1160 | 1160 | 1281 | 1236 |
| 1222 | 1174 | 1209 | 1321 | 1252 |
| 1236 | 1206 | 1243 | 1364 | 1357 |
| 1249 | 1224 | 1304 | 1432 | 1423 |
| 1278 | 1236 | 1356 | 1457 | 1456 |
| 1356 | 1259 | 1389 | 1481 | 1492 |
| 1370 | 1309 | 1434 | 1521 | 1577 |
| 1423 | 1356 | 1481 | 1569 | 1601 |
| 1456 | 1371 | 1561 | 1628 | 1643 |
| 1474 | 1422 | 1624 | 1720 | 1702 |
| 1491 | 1473 | 1654 | 3144 | 3342 |
| 1575 | 1497 | 1729 | 3288 | |
| 1620 | 1575 | 3159 | 3423 | |
| 1669 | 1622 | 3404 | | |
| 3294 | 1688 | 3498 | | |
| 3331 | 3195 | | | |
| 3479 | 3304 | | | |
| | 3472 | | | |
| | 3676 | | | |

- **Abbildung 1:**: **IR Spektrum der Verbindung der Formel (I) in der Modifikation I, II und III**
- **Abbildung 2:**: **IR Spektrum der Verbindung der Formel (I) in der Modifikation IV, V und als Triessigsäure Solvat**
- **Abbildung 3:**: **IR Spektrum der Verbindung der Formel (I) als Di-DMSO Solvat, DMF-Wasser Solvat und Monohydrat**
- **Abbildung 4:**: **IR Spektrum der Verbindung der Formel (I) als Dihydrat**
- **Abbildung 5:**: **Röntgendiffraktogramm der Verbindung der Formel (I) in der Modifikation I**
- **Abbildung 6:**: **Röntgendiffraktogramm der Verbindung der Formel (I) in der Modifikation II**
- **Abbildung 7:**: **Röntgendiffraktogramm der Verbindung der Formel (I) in der Modifikation III**
- **Abbildung 8:**: **Röntgendiffraktogramm der Verbindung der Formel (I) in der Modifikation IV**
- **Abbildung 9:**: **Röntgendiffraktogramm der Verbindung der Formel (I) in der Modifikation V**
- **Abbildung 10:**: **Röntgendiffraktogramm der Verbindung der Formel (I) als Triessigsäure Solvat**
- **Abbildung 11:**: **Röntgendiffraktogramm der Verbindung der Formel (I) als Di-DMSO Solvat**
- **Abbildung 12:**: **Röntgendiffraktogramm der Verbindung der Formel (I) als DMF-Wasser Solvat**
- **Abbildung 13:**: **Röntgendiffraktogramm der Verbindung der Formel (I) als Monohydrat**
- **Abbildung 14:**: **Röntgendiffraktogramm der Verbindung der Formel (I) als Dihydrat**

## Patentansprüche

1. Verbindung der Formel (I) in kristalliner Form der Modifikation I 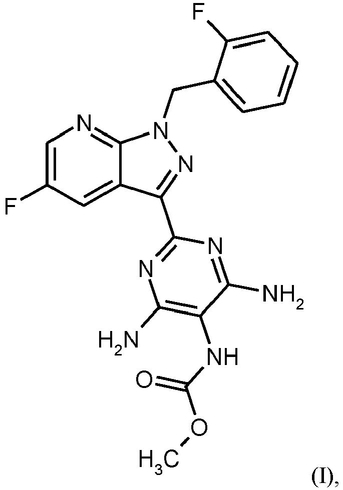 **dadurch gekennzeichnet, dass** das Röntgendiffraktogramm der Verbindung Peakmaxima des 2 Theta Winkels bei 5.9, 6.9, 16.2, 16.5, 24.1, 22.7, 24.7 zeigt.

2. Verbindung der Formel (I) in kristalliner Form der Modifikation I gemäß Anspruch 1 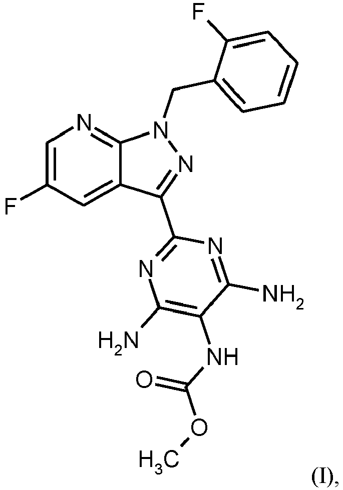 **dadurch gekennzeichnet, dass** das IR-Spektrum der Verbindung Bandenmaxima bei 1707, 1633, 1566, 1475, 1255, 1223 cm-1 zeigt.

3. Verfahren zur Herstellung der Verbindung der Formel (I) in kristalliner Form der Modifikation I gemäß Anspruch 1 oder 2, **dadurch gekennzeichnet, dass** die Verbindung der Formel (I), vorliegend als Di-Dimethylsulfoxid-Solvat in einem inerten Lösungsmittel bei einer Temperatur von 20°C - 120°C gerührt und die Verbindung der Formel (I) in der kristallinen Modifikation I isoliert wird.

4. Verbindung nach einem der Ansprüche 1 oder 2 zur Behandlung von Krankheiten.

5. Arzneimittel enthaltend eine Verbindung gemäß einem der Ansprüche 1 oder 2 in mehr als 90 Gewichtsprozente bezogen auf die Gesamtmenge der enthaltenen Verbindung der Formel (I).

6. Verbindung gemäß einem der Ansprüche 1 oder 2 zur Behandlung von Herz-Kreislauferkrankungen.

7. Verbindung der Formel (I) in kristalliner Form als Di-Dimethylsulfoxid-Solvat 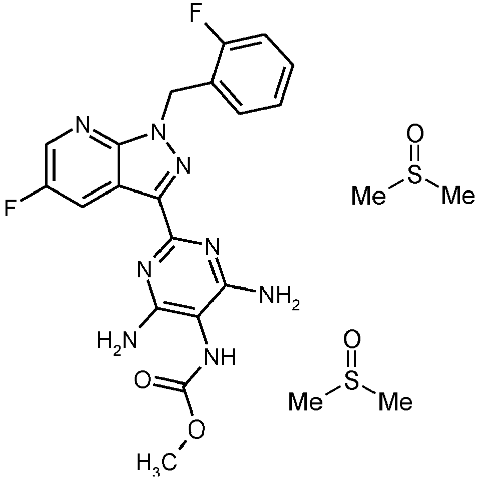 **dadurch gekennzeichnet, dass** das Röntgendiffraktogramm der Verbindung Peakmaxima des 2 Theta Winkels bei 12.0, 16.6, 17.8, 18.8, 20.3, 217 zeigt.

8. Verbindung der Formel (I) in kristalliner Form als Di-Dimethylsulfoxid-Solvat gemäß Anspruch 7 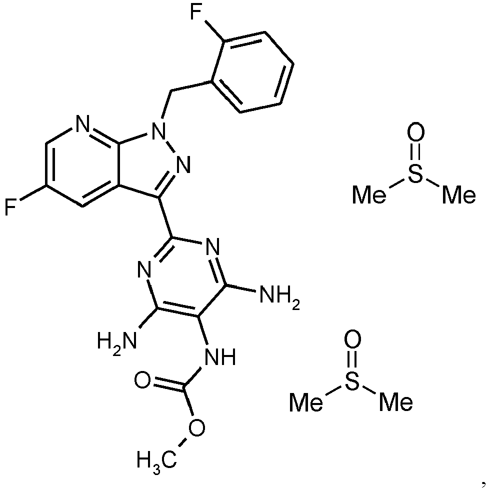 **dadurch gekennzeichnet, dass** das IR-Spektrum der Verbindung Bandenmaxima bei 1720, 1628, 1481, 1234, 1041, 1017 cm-1 zeigt.

## Claims

1. Compound of the formula (I) in crystalline form of the modification I 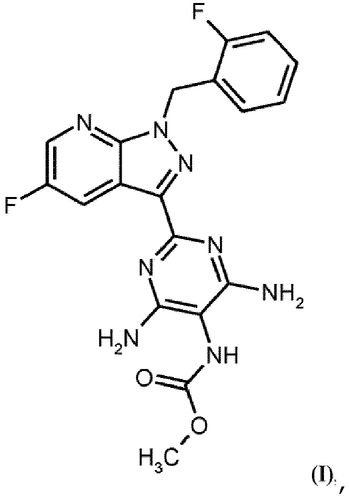 **characterized in that** the X-ray diffractogram of the compound shows peak maxima of the 2 theta angle at 5.9, 6.9, 16.2, 16.5, 24.1, 22.7, 24.7.

2. Compound of the formula (I) in crystalline form of the modification I according to Claim 1 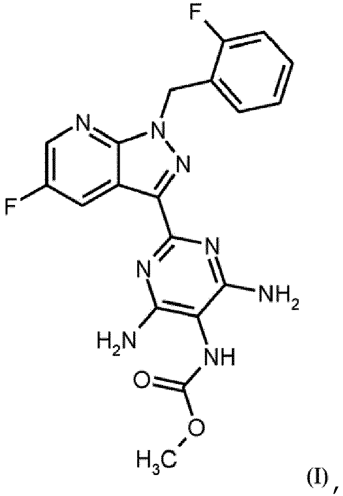 **characterized in that** the IR spectrum of the compound shows band maxima at 1707, 1633, 1566, 1475, 1255, 1233 cm⁻¹.

3. Method for preparing the compound of the formula (I) in crystalline form of the modification I according to Claim 1 or 2, **characterized in that** the compound of the formula (I) present as the di-dimethyl sulfoxide solvate is stirred in an inert solvent at a temperature of 20°C-120°C and the compound of the formula (I) is isolated in the crystalline modification I.

4. Compound according to either of Claims 1 or 2 for the treatment of medical conditions.

5. Medicament comprising a compound according to either of Claims 1 or 2 in a content of more than 90 percent by weight based on the total amount of the compound of the formula (I) present.

6. Compound according to either of Claims 1 or 2 for the treatment of cardiovascular conditions.

7. Compound of the formula (I) in crystalline form as the di-dimethyl sulfoxide solvate 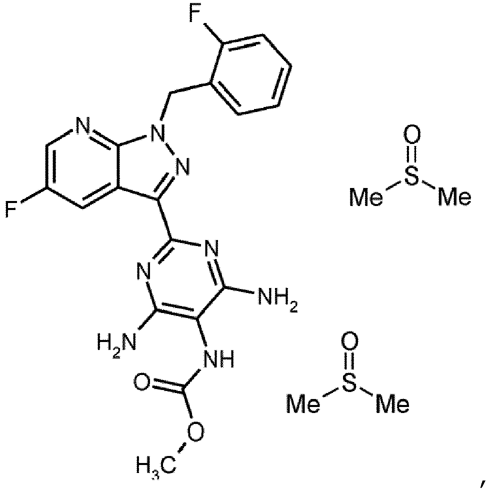 **characterized in that** the X-ray diffractogram of the compound shows peak maxima of the 2 theta angle at 12.0, 16.6, 17.8, 18.8, 20.3, 21.7.

8. Compound of the formula (I) in crystalline form as the di-dimethyl sulfoxide solvate according to Claim 7 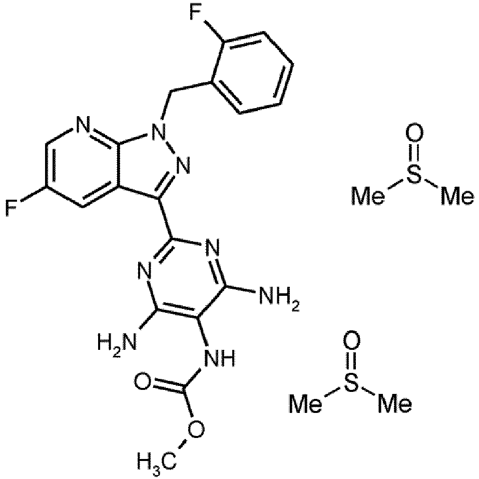 **characterized in that** the IR spectrum of the compound shows band maxima at 1720, 1628, 1481, 1234, 1041, 1017 cm⁻¹.

## Revendications

1. Composé de formule (I) sous forme cristalline de la modification I 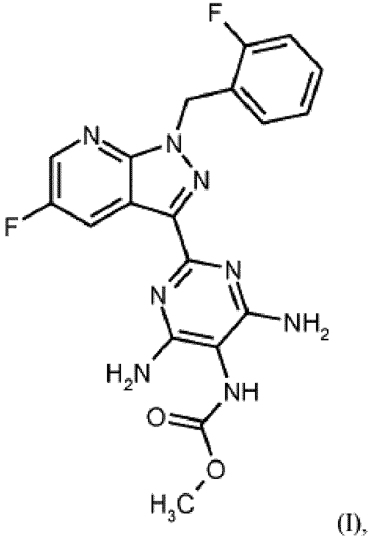 **caractérisé en ce que** le diffractogramme des rayons X du composé montre des maxima de pics de l'angle 2 thêta à 5,9, 6,9, 16,2, 16,5, 24,1, 22,7, 24,7.

2. Composé de formule (I) sous forme cristalline de la modification I selon la revendication 1 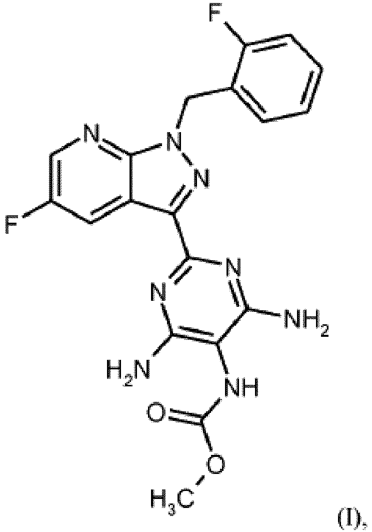 **caractérisé en ce que** le spectre IR du composé présente des maxima de bandes à 1707, 1633, 1566, 1475, 1255, 1223 cm⁻¹.

3. Procédé pour la préparation du composé de formule (I) sous forme cristalline de la modification I selon la revendication 1 ou 2, **caractérisé en ce que** le composé de formule (I), présent en tant que solvate double avec le diméthylsulfoxyde, est agité dans un solvant inerte à une température de 20 °C à 120 °C et le composé de formule (I) est isolé dans la modification cristalline I.

4. Composé selon l'une quelconque des revendications 1 et 2 pour le traitement de maladies.

5. Médicament contenant un composé selon l'une quelconque des revendications 1 et 2 à raison de plus de 90 pour cent en poids par rapport à la quantité totale du composé contenu de formule (I).

6. Composé selon l'une quelconque des revendications 1 et 2 pour le traitement de maladies cardiovasculaires.

7. Composé de formule (I) sous forme cristalline en tant que solvate double avec le diméthylsulfoxyde 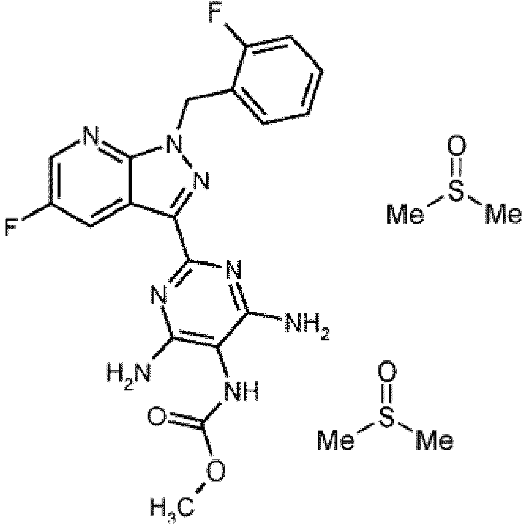 **caractérisé en ce que** le diffractogramme des rayons X du composé montre des maximas de pics de l'angle 2 thêta à 12,0, 16,6, 17,8, 18,8, 20,3, 21,7.

8. Composé de formule (I) sous forme cristalline en tant que solvate double avec le diméthylsulfoxyde selon la revendication 7 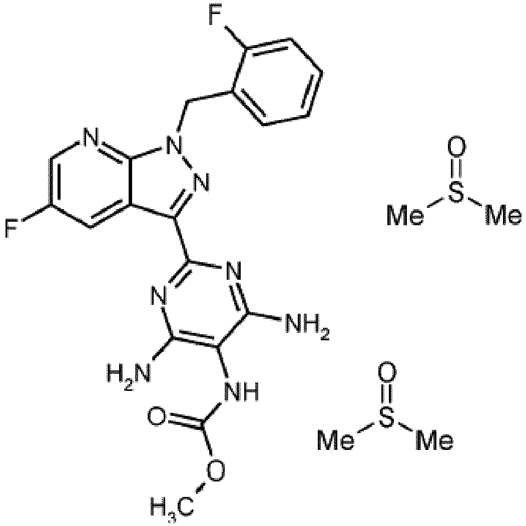 **caractérisé en ce que** le spectre IR du composé présente des maxima de bandes à 1720, 1628, 1481, 1234, 1041, 1017 cm⁻¹.
